(19) Europäisches Patentamt / European Patent Office / Office européen des brevets

(11) **EP 3 714 068 B1**

(12) **EUROPÄISCHE PATENTSCHRIFT**

(45) Veröffentlichungstag und Bekanntmachung des Hinweises auf die Patenterteilung:
**27.09.2023 Patentblatt 2023/39**

(21) Anmeldenummer: **18804614.8**

(22) Anmeldetag: **19.11.2018**

(51) Internationale Patentklassifikation (IPC):
**C12Q 1/6883** (2018.01)

(52) Gemeinsame Patentklassifikation (CPC):
**C12Q 1/6883;** C12Q 2600/112; C12Q 2600/118;
C12Q 2600/158

(86) Internationale Anmeldenummer:
**PCT/EP2018/081684**

(87) Internationale Veröffentlichungsnummer:
**WO 2019/101665 (31.05.2019 Gazette 2019/22)**

(54) **VERWENDUNG DES IL-6 GENEXPRESSIONSLEVELS ZUR EINORDNUNG EINES PROBANDEN IN RISIKOGRUPPEN BEI DER PROGNOSE ODER DIAGNOSE EINER DIABETES MELLITUS TYP II ERKRANKUNG**

USE OF THE IL-6 GENE EXPRESSION LEVEL TO CLASSIFY A PROBAND INTO A RISK GROUP FOR THE PROGNOSIS OR DIAGNOSIS OF DIABETES MELLITUS TYPE 2 DISEASE

UTILISATION DU NIVEAU D'EXPRESSION DU GÈNE IL-6 POUR CLASSER UN SUJET DANS DES GROUPES DE RISQUE LORS DU PRONOSTIC OU DU DIAGNOSTIC D'UN DIABÈTE SUCRÉ DE TYPE II

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.11.2017 DE 102017127858**

(43) Veröffentlichungstag der Anmeldung:
**30.09.2020 Patentblatt 2020/40**

(73) Patentinhaber: **Lipozyt Marker GmbH**
**28357 Bremen (DE)**

(72) Erfinder:
• **JENSEN, Uwe**
  **28357 Bremen (DE)**
• **HIERNEIS, Wolfgang**
  **20146 Hamburg (DE)**
• **KLEMKE, Markus**
  **28777 Bremen (DE)**
• **THIES, Helge Wilhelm**
  **28359 Bremen (DE)**

(74) Vertreter: **Eisenführ Speiser**
**Patentanwälte Rechtsanwälte PartGmbB**
**Am Kaffee-Quartier 3**
**28217 Bremen (DE)**

(56) Entgegenhaltungen:
• **ALMURAIKHY SHAMMA ET AL: "Interleukin-6 induces impairment in human subcutaneous adipogenesis in obesity-associated insulin resistance", DIABETOLOGIA, SPRINGER, BERLIN, DE, Bd. 59, Nr. 11, 24. Juni 2016 (2016-06-24) , Seiten 2406-2416, XP036274061, ISSN: 0012-186X, DOI: 10.1007/S00125-016-4031-3 [gefunden am 2016-06-24]**
• **Krishana Gopal ET AL: "THE ROLE OF TNF-ALPHA AND IL-6 LEVEL WITH IR IN TYPE-II DIABETES MELLITUS. ORIGINAL RESEARCH PAPER", , 1. Februar 2017 (2017-02-01), XP055533783, Gefunden im Internet: URL:https://wwjournals.com/index.php/ijar/article/view/15562 [gefunden am 2018-12-12]**
• **SMITHA UPADHYAYA ET AL: "Adiponectin and IL-6 : Mediators of inflammation in progression of healthy to type 2 diabetes in Indian population", ADIPOCYTE, Bd. 3, Nr. 1, 8. Oktober 2013 (2013-10-08) , Seiten 39-45, XP055533648, ISSN: 2162-3945, DOI: 10.4161/adip.26553**

- HUANG HUIMIN ET AL: "HMGA2, a driver of inflammation, is associated with hypermethylation in acute liver injury", TOXICOLOGY AND APPLIED PHARMACOLOGY, Bd. 328, 11. Mai 2017 (2017-05-11), Seiten 34-45, XP085066374, ISSN: 0041-008X, DOI: 10.1016/J.TAAP.2017.05.005
- TETSUJI SHINOHARA: "Interleukin-6 as an Independent Predictor of Future Cardiovascular Events in Patients with Type-2 Diabetes without Structural Heart Disease", JOURNAL OF CLINICAL & EXPERIMENTAL CARDIOLOGY, Bd. 03, Nr. 09, 1. Januar 2012 (2012-01-01), XP055534224, DOI: 10.4172/2155-9880.1000209
- Emma Ahlqvist ET AL: "Clustering of adult-onset diabetes into novel subgroups guides therapy and improves prediction of outcome", bioRxiv, 8. September 2017 (2017-09-08), XP55532289, DOI: 10.1101/186387 Gefunden im Internet: URL:https://www.biorxiv.org/content/biorxiv/early/2017/09/08/186387.full.pdf
- Mia Louise Westerlund: "Classication with Kohonen Self-Organizing Maps", , 24. April 2005 (2005-04-24), XP55532367, Gefunden im Internet: URL:https://notendur.hi.is/benedikt/Courses/Mia_report2.pdf [gefunden am 2018-12-10]

**Beschreibung**

Die Erfindung betrifft die Verwendung des relativen Wertes des Genexpressionslevels des Gens *IL-6* bei der Prognose oder der Diagnose einer Diabetes mellitus Typ II Erkrankung eines Probanden. Sie betrifft ferner ein Verfahren zur Prognose und/oder die Diagnose einer Diabetes mellitus Typ II Erkrankung zur Einordnung eines Probanden in Risikogruppen, wobei das Genexpressionslevel des Gens *IL-6* bestimmt wird und nachfolgend die Einordnung des Probanden unter Berücksichtigung dieses Genexpressionslevels in Risikogruppen erfolgt.

In den letzten Jahrzehnten stieg der Anteil der an Übergewicht und Adipositas leidenden Personen in den westlichen Industrienationen stark an. Derzeit sind etwa ein Drittel aller Menschen in Wohlstandgesellschaften übergewichtig, es werden für die Zukunft aber weitere Zunahmen dieses Anteils prognostiziert. So könnten bereits im Jahr 2030 45 Prozent der deutschen Bevölkerung an Adipositas leiden. Für Betroffene stellt die Fettleibigkeit ein hohes gesundheitliches Risiko dar, da sie verschiedene Folgekrankheiten wie beispielsweise Diabetes mellitus Typ II nach sich ziehen. Damit geht eine erhebliche Verminderung der Lebenserwartung einher. Das Fettgewebe, das bei adipösen Personen im Überschuss vorhanden ist, dient dabei aber nicht nur als reiner Speicher für Energiereserven.

Neben seiner Funktion als Speicherorgan stellt das Fettgewebe auch das größte endokrine Organ dar, das zahlreiche Botenstoffe freisetzt. Insbesondere ein Überschuss an viszeralem Fettgewebe bedingt eine Erhöhung des Risikos für mit Adipositas einhergehende Erkrankungen mit entzündlicher Komponente. Hierzu zählt beispielsweise Typ-2-Diabetes. Adipozyten spielen eine wichtige Rolle im Lipid- und Glukosestoffwechsel und tragen zur Regulation und der Beibehaltung eines Gleichgewichts des Blutzuckerspiegels bei. Metabolische Dysfunktionen des Fettgewebes können ursächlich für die Entwicklung einer Insulinresistenz und die Ausbildung eines Typ-2-Diabetes sein.

Bei Adipositas nimmt nicht nur die Fettmasse zu, sondern es werden auch vermehrt T-Lymphozyten und Makrophagen im Fettgewebe rekrutiert. Diese setzen vermehrt Botenstoffe frei, die man als Zytokine bezeichnet. Eine gesteigerte Freisetzung proinflammatorischer Zytokine kann einen als "Metainflammation" bezeichneten Zustand herbeiführen. Darunter versteht man eine anhaltende, unkontrollierte Entzündungsreaktion, die ein Schlüsselereignis zahlreicher Erkrankungen ist, darunter auch Stoffwechselerkrankungen, die im Zusammenhang mit Adipositas stehen. Die von den Zytokinen ausgelösten Signalkaskaden können einen negativen Einfluss auf das Insulinsystem ausüben und Fehlfunktionen in Zielgeweben wie der Leber, den Skelettmuskeln und dem Fettgewebe auslösen (Mauer J, Denson JL, Brüning JC. 2015. Versatile functions for IL-6 in metabolism and cancer. Trends Immunol. 36: 92-101).

Eines der bedeutendsten inflammatorischen Zytokine im Zusammenhang mit Adipositas und Diabetes ist Interleukin-6 (IL-6), das von T-Zellen und Makrophagen sezerniert wird. Etwa ein Drittel des im Blutplasma zirkulierenden IL-6 stammt aus Fettgeweben. Die IL-6-Menge im Plasma korreliert positiv mit einer Adipositas, d. h. im Plasma fettleibiger Menschen werden i. d. R. höhere IL-6-Konzentrationen angetroffen. Einer vermehrten IL-6-Sekretion bei Adipositas wird ein Beitrag an der metabolischen Dysfunktion zugeschrieben. Auch bei Patienten mit Typ-2-Diabetes lassen sich erhöhte IL-6-Level im Blutplasma nachweisen, jedoch wird die Rolle des IL-6 im Zusammenhang mit einer Insulinresistenz kontrovers diskutiert (Kern PA, Ranganathan S, Li C, Wood L, Ranganathan G. 2001. Adipose tissue tumor necrosis factor and interleukin-6 expression in human obesity and insulin resistance. Am J Physiol Endocrinol Metab. 280: E745-E751; Ouchi N, Parker JL, Lugus JJ, Walsh K. 2011. Adipokines in inflammation and metabolic disease. Nat Rev Immunol. 11: 85-97).

IL-6 kann eine Signalkaskade in Zellen auslösen, die über einen membranständigen Rezeptor (IL-6R) verfügen. Allerdings besitzen nur wenige Zelltypen diesen Rezeptor. IL-6 kann aber auch von einem löslichen Rezeptor (sIL-6R) gebunden werden, woraufhin dieser Komplex an das Glykoprotein gp130 binden kann, den alle Zellen besitzen, wodurch das Spektrum der IL-6-Zielzellen stark erweitert wird. Diese als trans-Signalweg bezeichnete Variante vermittelt eher pro-inflammatorische Antworten, wohingegen der klassische über IL6R vermittelte Signalweg eher mit anti-inflammatorischen Reaktionen in Verbindung steht (Scheller J, Chalaris A, Schmidt-Arras D, Rose-John S. 2011. The proand anti-inflammatory properties of the cytokine interleukin-6. Biochim Biophys Acta. 1813: 878-888.; Rose-John S. 2012. IL-6 trans-signaling via the soluble IL-6 receptor: importance for the pro-inflammatory activities of IL-6. Int J Biol Sci. 8: 1237-1247).

Bei einer fettreichen Ernährung spielt IL-6 aus dem Fettgewebe eine Rolle bei der vermehrten SOCS3-Expression in der Leber. Dieses Protein wiederum ist mitverantwortlich für eine Insulinresistenz der Leber (Sabio G, Das M, Mora A, Zhang Z, Jun JY, Ko HJ, Barrett T, Kim JK, Davis RJ. 2008. A stress signaling pathway in adipose tissue regulates hepatic insulin resistance. Science. 322: 1539-1543).

Andererseits wurde aber auch gezeigt, dass ein gesteigerter IL-6-Spiegel die Ausschüttung des Hormons GLP-1 (Glucagon-like peptide 1) aus L-Zellen des Darms anregt, welches wiederum die Insulinsekretion des Pankreas steigert. Es dient also der Kommunikation zwischen insulinsensitiven Geweben, den L-Zellen des Darms und den Inselzellen des Pankreas (Ellingsgaard H, Hauselmann I, Schuler B, Habib AM, Baggio LL, Meier DT, Eppler E, Bouzakri K, Wueest S, Muller YD, Hansen AM, Reinecke M, Konrad D, Gassmann M, Reimann F, Halban PA, Gromada J, Drucker DJ, Gribble FM, Ehses JA, Donath MY. 2011. Interleukin-6 enhances insulin secretion by increasing glucagon-like peptide-1 secretion from L cells and alpha cells. Nat Med. 17: 1481-1489). Somit übt IL-6 vielfältige Funktionen aus, die außerdem bislang nicht vollständig aufgeklärt werden konnten. IL-6 in Komination mit Adiponektin wurde as Entzündungsmediator in der Progression von gesunden zu T2D Patienten in indischen Probanden bestimmt (Adipocyte (2014) 3(1) 39-45).

Zur Vorhersage des persönlichen Risikos, eine mit Adipositas assoziierte Folgeerkrankung zu erleiden, fehlen derzeit geeignete Parameter. Eine frühzeitige Prognose ist aber von besonderer Bedeutung, um rechtzeitig geeignete Maßnahmen ergreifen zu können, um eine drohende Erkrankung abwenden, ihren Eintritt hinauszögern oder ihren Verlauf abmildern zu können. Ist eine Folgeerkrankung der Fettleibigkeit bereits eingetreten, ist mit einem geringeren Erfolg therapeutischer Maßnahmen sowie einem höheren Verlust an Lebensqualität zu rechnen. Wäre man in der Lage, lange vor Ausbruch einer Krankheit ein hohes individuelles Erkrankungsrisiko zu erkennen, ließen sich bessere Therapieerfolge erzielen. Daher ist es das Ziel dieser Erfindung, Probanden mit Hilfe der Genexpression im subkutanen Fettgewebe in verschiedene Subgruppen klassifizieren zu können, denen bestimmte Stoffwechselzustände und Erkrankungsrisiken zugeordnet werden können.

Vor diesem Hintergrund war es Aufgabe der vorliegenden Erfindung eine neue Möglichkeit anzugeben, mittels derer eine gegenüber dem Stand der Technik andere bzw. zusätzlich ausdifferenzierte Prognose und/oder Diagnose möglich ist.

Erfindungsgemäß gelöst wird diese Aufgabe durch Verwendung des relativen Wertes des Genexpressionslevels Gens für *IL-6* bei der Prognose und/oder der Diagnose einer Diabetes mellitus Typ II-Erkrankung eines Probanden, wobei der Proband in eine von wenigstens vier Risikogruppen eingeordnet wird.

"IL-6" im Sinne dieses Textes ist das Interleukin-6 bzw. dessen Gen bzw. die zugehörige mRNA und/oder Teile dieses Proteins bzw. Gens (bzw. dessen mRNA) bevorzugt wenigstens eine Aminosäurenkette $\geq$ 7 Aminosäuren, weiter bevorzugt $\geq$ 15 Aminosäuren und besonders bevorzugt $\geq$ 20 Aminosäuren bzw. eine Nucleinsäurenkette von $\geq$ 20 Nucleinsäuren, weiter bevorzugt $\geq$ 40 Nucleinsäuren und besonders bevorzugt $\geq$ 55 Nucleinsäuren ggf. pro Strang. Interleukin-6 ist ein Zytokin, das sowohl in Entzündungsreaktionen als auch bei der Reifung von B-Lymphozyten eine Rolle spielt. Darüber hinaus wurde nachgewiesen, dass es sich um einen endogenen, entzündlich wirkenden Stoff, ein sogenanntes Pyrogen handelt, das beim Vorliegen von Autoimmunerkrankungen oder Infektionen hohes Fieber auslösen kann. Das Protein wird vorwiegend an Orten akuter oder chronischer Inflammation erzeugt, von wo aus es ins Serum sezerniert wird und über den Interleukin-6-Rezeptor alpha eine inflammatorische Reaktion auslöst. Interleukin-6 ist in verschiedene entzündungsassoziierte Krankheitszustände involviert, darunter eine Prädisposition für Diabetes mellitus oder die systemische juvenile idiopathische Arthritis (Still-Syndrom). Das humane IL-6-Gen ist in der chromosomalen Bande 7p15.3 lokalisiert und besteht aus sechs Exons. Das IL-6-Präkursorprotein besteht aus 212 Aminosäuren. Nach Abspaltung eines 28 Aminosäuren langen Signalpeptids besitzt das reife Interleukin-6 eine Länge von 184 Aminosäuren (Hirano T, Yasukawa K, Harada H, Taga T, Watanabe Y, Matsuda T, Kashiwamura S, Nakajima K, Koyama K, Iwamatsu A, et al., 1986. Complementary DNA for a novel human interleukin (BSF-2) that induces B lymphocytes to produce immunoglobulin. Nature 324: 73-76).

Die Bestimmung des Wertes für das relative Genexpressionslevel im Sinne der vorliegenden Erfindung kann auf jede dem Fachmann bekannte Weise erfolgen. Bevorzugt ist eine Bestimmung des Genexpressionslevels auf mRNA-Ebene oder auf Proteinebene. Bevorzugt ist dabei die mRNA-Ebene.

Bei der vorliegenden Erfindung werden relative Genexpressionslevel bestimmt. Sofern nachfolgend lediglich von "Genexpressionslevel" gesprochen wird, handelt es sich - sofern nicht anders angemerkt - stets um relative Genexpressionslevel. Die relativen Genexpressionslevel werden dabei bevorzugt durch Bestimmung des Genexpressionslevels des zu untersuchenden Gens im Verhältnis zum Expressionslevels eines housekeeping Gens, bevorzugt ausgewählt aus der Gruppe bestehend aus *HPRT,* 18S rRNA, *GAPDH, GUSB, PBGD, B2M, ABL, RPLP0*, wobei ganz besonders *HPRT* bevorzugt ist, bestimmt.

Der Begriff "Prognose" bedeutet im Sinne dieses Textes eine Vorhersage einer erhöhten Wahrscheinlichkeit der Entwicklung oder des Auftretens eines klinischen Zustandes oder einer Krankheit.

Der Begriff "Diagnose" einer Krankheit bedeutet im Sinne dieses Textes, dass eine Krankheit, die bereits klinische Symptome zeigt, identifiziert und/oder bestätigt wird.

Diabetes mellitus Typ II wird nachfolgend im Text auch mit T2D abgekürzt.

"Proband" im Sinne der vorliegenden Anmeldung sind Mensch und Tier, wobei bevorzugt Menschen gemeint sind.

"Risikogruppen" im Sinne dieses Textes sind solche Gruppen, die durch geeignete Unterscheidungsmerkmale voneinander getrennt werden können und jeweils ein gemeinsames erhöhtes oder nicht erhöhtes Risiko in Bezug auf die Entwicklung oder das Vorhandensein einer Krankheit insbesondere T2D besitzen. Zudem können sich Risikogruppen zusätzlich durch weitere physiologische Unterschiede voneinander unterscheiden, was ggf. therapeutische oder prophylaktische Relevanz besitzt.

Überraschenderweise hat sich herausgestellt, dass es aufgrund der erfindungsgemäßen Verwendung möglich ist, bei Prognose und/oder Diagnose einer T2D-Erkrankung wenigstens vier Risikogruppen zu unterscheiden. Diese Unterscheidung hilft beim Auffinden geeigneter Therapien für das jeweilige Individuum.

Ein solcher Therapieansatz könnte beispielsweise sein, dass in Abhängigkeit von der IL-6-Expression innerhalb der T2D-Erkrankten Medikamente gegeben werden, die den IL-6-Spiegel beeinflussen oder die Bindung von IL-6 an dessen Rezeptor verhindert. Der monoklonale Antikörper Tocilizumab inhibiert z.B. die Bindung von IL-6 an dessen Rezeptor und könnte somit bei hoher IL-6-Expression bei Typ-2-Diabetikern eine effektive Behandlungsoption darstellen.

Der Schutzbereich der Erfindung wird durch die Ansprüche definiert und betrifft ein Verfahren zur Prognose und/oder Diagnose einer Diabetes mellitus Typ II-Erkrankung, umfassend die Schritte:

a) Bestimmen des Genexpressionslevels des Genes *IL-6* in der Probe und

b) Einordnen des Probanden unter Berücksichtigung des Genexpressionslevels des Genes IL- 6 in eine von wenigstens vier Risikogruppen.

Wie bereits bei der erfindungsgemäßen Verwendung beschrieben, ergibt sich überraschenderweise durch das Einbeziehen des relativen Genexpressionslevels des Genes *IL-6* bei der Einordnung in eine von wenigstens vier Risikogruppen die Möglichkeit, innerhalb der Probanden (z. B. der bereits an Diabetes mellitus Typ II erkrankten Probanden) eine weitere Differenzierung festzustellen, die wiederum für therapeutische Zwecke genutzt werden kann.

Beim Einordnen der Probanden in Schritt b) wird erfindungsgemäss ferner der Genexpressionslevel von *HMGA2*, berücksichtigt.

Eine Möglichkeit eine Fehlentwicklung im Hinblick auf die Insulinresistenz und - produktion frühzeitig zu diagnostizieren liegt u.a. im Fettgewebe, das als Langzeitspeicher für Fette und Glukose eine Fehlernährung über einen sehr langen Zeitraum abpuffern kann. Vermutlich ist ein dysfunktionales Fettgewebe ein entscheidendes Element für die Entstehung von T2D, sowohl bei Übergewichtigen/Adipositas-Erkrankten als auch Normalgewichtigen. Verschiedene Studienergebnisse zeigen, dass Fettgewebe vermehrt als endokrines Organ, welches aktiv in physiologische Vorgänge eingreift bzw. diese steuert, anzusehen ist. Die vom Fettgewebe sezernierten Stoffe, Adipokine genannt, sind u.a. mit der Insulinsensitivität und -resistenz, der Fortpflanzung, der Inflammation und dem Knochenwachstum, sowie mit immunologischen Prozessen und dem Fettsäuremetabolismus assoziiert. Ein mit der Prädisposition für die Entwicklung von T2D verbundenes Adipokin ist Adiponektin (Szmitko PE, Teoh H, Stewart DJ, Verma S (2007) Adiponectin and cardiovascular disease: state of the art? Am J Physiol Heart Circ Physiol. 292:H1655-63).

Das humane Adiponektin wird vom *APM1/ACoC/ACRP30/GBP28/AoIPOQ-Gen* (Accession ID: D45371) kodiert, welches in der chromosomalen Bande 3q27 lokalisiert ist. Es enthält drei Exons die in einer 17kb großen Region liegen. Die Exons eins und zwei sind 76 bzw. 222 bp groß und sind durch Intron eins mit der Größe von 10,3 kb getrennt. Das Exon drei umfasst ungefähr 4,28 kb. Die Translation beginnt im Exon zwei und endet im Exon drei und lässt somit Exon eins und Teile des Exons drei untranslatiert. Das 30-kDa große Adiponektin-Protein wird hauptsächlich von Adipozyten produziert und sezerniert. Adiponektin besteht aus einer carboxyterminalen globulären Domäne und einer kollagenen Domäne im aminoterminalen Ende. Im Blutplasma kommt Adiponektin als vollständiges, 244 Aminosäuren langes, Protein und als proteolytisches Spaltproduktfragment, auch globuläres Adiponektin genannt, vor. Isoformen des Adiponektins entstehen aufgrund unterschiedlicher Verknüpfungen der globulären und kollagenen Domänen. Die Wirkung von Adiponektin auf die Körperzellen wird über die AdipoR1 und AdipoR2 Rezeptoren vermittelt. In erster Linie sind AdipoR1 im Skelettmuskel und AdipoR2 in Leberzellen zu finden. Weitere Studien lassen allerdings vermuten, dass AdipoR1 und AdipoR2 auch in Kardiomyozyten, Osteoblasten und β-Zellen des Pankreas exprimiert werden. Im Lipid- und Glukosemetabolismus spielt Adiponektin eine eminente Rolle. Es bewirkt eine Veränderung der Insulinsensitivität über Aktivierung der 5'Adenosinmonophosphataktivierte Proteinkinase (AMPK) und verbessert die Insulinresistenz mittels Steigerung der Fettsäureoxidation und Unterdrückung der Glukoneogenese in der Leber. Ein Mangel an Adiponektin ist neben dem erhöhten Risiko für Diabetes und einer diabetischen Angiopathie, auch mit einem erhöhten Risiko für Herzinfarkte und Schlaganfälle verbunden.

"Adiponektin" im Sinne dieses Textes ist dementsprechend das Adiponektin Protein bzw. dessen Gen bzw. die zugehörige mRNA und/oder Teile dieses Proteins bzw. Gens (bzw. dessen mRNA) bevorzugt wenigstens eine Aminosäurenkette ≥ 7 Aminosäuren, weiter bevorzugt ≥ 15 Aminosäuren und besonders bevorzugt ≥ 20 Aminosäuren bzw. eine Nucleinsäurenkette von ≥ 20 Nucleinsäuren, weiter bevorzugt ≥ 40 Nucleinsäuren und besonders bevorzugt ≥ 55 Nucleinsäuren ggf. pro Strang. Adiponektin ist ein wichtiges Adipokin, das an der Kontrolle des Fettstoffwechsels und der Insulinsensitivität beteiligt ist und einen direkten anti-diabetischen, anti-atherogenen und entzündungshemmenden Einfluss hat. Es stimuliert die AMPK-Phosphorylierung und -Aktivierung in der Leber und dem Skelettmuskel, wodurch die Glukoseverwertung und die Fettsäureverbrennung erhöht werden. Im Plasma zirkulieren vor allem drei Hauptkomplexe, ein niedermolekulares Trimer (LMW), ein mittelmolekulares Hexamer (MMW) und ein hochmolekularer Komplex (HMW).

"HMGA2" im Sinne dieses Textes ist das High Mobility Group AT-Hook Protein 2 (HMGA2) bzw. dessen Gen bzw. die zugehörige mRNA und/oder Teile dieses Proteins bzw. Gens (bzw. dessen mRNA) bevorzugt wenigstens eine Aminosäurenkette ≥ 7 Aminosäuren, weiter bevorzugt ≥ 15 Aminosäuren und besonders bevorzugt ≥ 20 Aminosäuren bzw. eine Nucleinsäurenkette von ≥ 20 Nucleinsäuren, weiter bevorzugt ≥ 40 Nucleinsäuren und besonders bevorzugt ≥ 55 Nucleinsäuren ggf. pro Strang. HMGA2 ist ein Transkriptionsfaktor der die Regulation der Genexpression beeinflusst und zur Gruppe der High Mobility Group A-Proteine (HMGA-Proteine) gehört. Die HMGA-Proteine sind Chromatin-assoziierte, säurelösliche Nicht-Histon-Proteine, die an sequenzunabhängige, spezifische Motive der DNA binden. Als architektonische Transkriptionsfaktoren erhöhen bzw. inhibieren sie über strukturelle Änderungen der Chromatinorganisation die Bindungsfähigkeit weiterer Transkriptionsfaktoren. Das humane HMGA2-Gen ist in der chromosomalen Region 12q14~15 lokalisiert und besteht aus fünf Exons, die sich über einen ≥160kb langen Bereich erstrecken. Es codiert ein 109 Aminosäuren langes Protein, dessen molekulare Masse 12 kDa beträgt. Das HMGA2-Protein ist durch drei stark konservierte DNA-bindende Domänen, die sogenannten AT-Hooks und eine saure negativ geladene C-terminale Domäne gekennzeichnet.

"PPAR-gamma" im Sinne dieses Textes ist der Peroxisom-Proliferator-aktivierte Rezeptor gamma (PPAR-gamma) bzw. dessen Gen bzw. die zugehörige mRNA und/oder Teile dieses Proteins bzw. Gens (bzw. dessen mRNA) bevorzugt wenigstens eine Aminosäurenkette ≥ 7 Aminosäuren, weiter bevorzugt ≥ 15 Aminosäuren und besonders bevorzugt ≥ 20 Aminosäuren bzw. eine Nucleinsäurenkette von ≥ 20 Nucleinsäuren, weiter bevorzugt ≥ 40 Nucleinsäuren und besonders bevorzugt ≥ 55 Nucleinsäuren ggf. pro Strang. PPAR-gamma ist ein Liganden-bindender nukleärer Transkriptionsfaktor der PPAR-Unterfamilie, der zur Gruppe der nukleären Hormonrezeptoren gehört. Über Heterodimerisation mit dem Retinoid X Rezeptor α (RXRα) aktiviert PPAR-gamma die Transkription verschiedener Gene. Das humane *PPAR-gamma-Gen* ist in der chromosomalen Bande 3p25 lokalisiert und besteht aus 11 Exons. Das humane PPAR-gamma-Gen codiert für 2 Isoformen, die jeweils ein 477 und ein 505 Aminosäuren langes Protein darstellen.

Es hat sich dabei herausgestellt, dass bei der erfindungsgemäßen Verwendung bzw. bei dem erfindungsgemäßen Verfahren die Kombination der Daten für *IL-6* mit denen der genannten bevorzugten Gene zu besonders sicheren Gruppenunterscheidungen innerhalb der Risikogruppen führen.

Bevorzugt ist ein erfindungsgemäßes Verfahren bzw. eine erfindungsgemäße Verwendung, wobei für die Eingruppierung in Risikogruppen (in Schritt b)) ferner eines oder mehrere Merkmale des Probanden ausgewählt aus der Gruppe bestehend aus Alter, BMI, Größe, Gewicht, Geschlecht, Bauch- & Hüftumfang, Körperfettanteil, Muskelmasse, Total Body Water (TBW), Blutdruck, Raucherstatus, Bluthochdruck, Einnahme blutdrucksenkender Medikamente berücksichtigt wird.

Somit ist es möglich, mittels der Berücksichtigung weiterer Merkmale der Probanden eine zusätzliche Sicherheit der Aufspaltung innerhalb der Risikogruppen zu erzeugen. Teilweise ist es selbstverständlich auch möglich, mit jedem zusätzlichen Marker (der Berücksichtigung eines weiteren Merkmals) die Aufspaltung der Risikogruppen noch weiter zu verfeinern.

In diesem Sinne ist es erfindungsgemäß bevorzugt, dass bei der Eingruppierung in die Risikogruppen, insbesondere in Schritt b) ferner einer oder mehrere Blutwerte des Probanden ausgewählt aus der Gruppe bestehend aus Gesamtcholesterin, Triglyceride, HbA1c, HDL-Cholesterin, non-HDL-Cholesterin, LDL-Cholesterin, CRP, Blutzucker, Nüchternblutzucker und präprandialer Blutzucker und postprandialer Blutzucker berücksichtigt wird.

Es ist bekannt, dass mittels der Blutwerte relevante Aussagen über den Status hinsichtlich von T2D gemacht werden können.

Mithilfe des erfindungsgemäßen Einsatzes von *IL-6* lassen sich in Kombination mit den zusätzlichen Markern/Blutparametern weitere Gruppenausdifferenzierungen erzeugen.

Die nachfolgende Tabelle gibt Hinweise zu typischen Werten für Blutparameter mit einem Normreferenzbereich laut Leitlinie der Deutschen Diabetes Gesellschaft von 2012 sowie der ESC/EAS Guidelines for the Management of Dyslipidaemias von 2016:

**Tabelle 1**

| Parameter | Norm-Referenzbereich (mg/dL) | In T2D-Gruppe | In Nicht-T2D-Gruppe |
|---|---|---|---|
| Triglyceride | < 150 | Erhöht | Nicht-erhöht /niedrig |
| Cholesterin, ges* | **<20 Jahre:** <170<br>**20-30 Jahre:** <200<br>**30-40 Jahre:** <220<br>**>40 Jahre:** <240 | Erhöht | Nicht-erhöht /niedrig |
| HDL-Cholesterin | Männer > 40<br>Frauen > 48 | Erniedrigt | Erhöht |

(fortgesetzt)

| Parameter | Norm-Referenzbereich (mg/dL) | In T2D-Gruppe | In Nicht-T2D-Gruppe |
|---|---|---|---|
| non- HDL-Cholesterin + | **-Sehr hohes Risiko:** < 100<br>**-hohes Risiko:** <130<br>**-Niedriges bis moderates Risiko:** <145 | Erhöht | Nicht-erhöht /niedrig |
| LDL-Cholesterin + | **-Sehr hohes Risiko:** <70<br>**-hohes Risiko:** <100<br>**-Niedriges bis moderates Risiko:** <115 | Erhöht | Nicht-erhöht /niedrig |
| CRP | < 5,0 mg/L | Erhöht | Nicht-erhöht /niedrig |
| Nüchtern-Glukose (venöses Plasma) | < 100 | Erhöht | Nicht-erhöht /niedrig |
| 2h-Glukosewert (venöses Plasma) | <120 | Erhöht | Nicht-erhöht /niedrig |
| Präprandiale Glukose (venöses Plasma) | < 100 | Erhöht | Nicht-erhöht /niedrig |
| HbA1c | < 5,7% | Erhöht | Nicht-erhöht /niedrig |

\* Der durchschnittliche Gesamtcholesterinspiegel der Altersgruppe zwischen 35 und 65 Jahren in Deutschland liegt bei etwa 236 mg/dl, die Standardabweichung bei ±46 mg/dl.

+ Die Abstufung basiert auf sehr hohem, hohem und niedrigen bis moderatem Risiko für kardiovaskuläre Erkrankungen (s. aktuelle Leitlinien von European Society of Cardiology (ESC) and European Atherosclerosis Society (EAS) für 2016: ESC/EAS Guidelines for the Management of Dyslipidaemias). Das Risiko kann mit einem SCORE (Systematic Coronary Risk Estimation) errechnet werden. Die verbreitesten Score-Systeme sind der ESC-SCORE und der PROCAM-SCORE (Prospective Cardiovascular Munster Study).

Hierbei kann die Differenzierung des Status der jeweiligen Blutwerte erfindungsgemäß in Kombination mit anderen Markern zur zusätzlichen Aufspaltung innerhalb der Risikogruppen bei geeigneter Auswertung (vgl. auch unten) führen.

Bevorzugt ist ein erfindungsgemäßes Verfahren, wobei die Probe aus Fettgewebe gewonnen wurde.

Dabei ist unter der "Probe aus Fettgewebe" diejenige Probe zu verstehen, die die Werte für wenigstens ein Genexpressionslevel, bevorzugt für alle Genexpressionslevel liefert, die für Schritt c) verwendet werden. Selbstverständlich können die Blutwerte nicht aus Fettgewebe gewonnen werden.

Bevorzugt ist dabei für das bevorzugte erfindungsgemäße Verfahren, dass die Probe aus Fettgewebe durch Punktion des subkutanen abdominalen Fettgewebes gewonnen wurde.

Durch fächerförmige Punktierungen unter Sog lassen sich besonders gut Zellen und Zellverbände gewinnen, die eine molekulargenetische Analyse ermöglichen. Das fächerförmige Vorgehen bei der Punktion vermindert zum einen eine Verklebung/Verstopfung der Kanülenspitze mit Fettzellen, zum anderen erhält man Zellen aus verschiedenen Regionen des betreffenden Fettgewebes und hat somit einen repräsentativen Querschnitt der Verteilung unterschiedlicher Fettgewebszelltypen.

Besonders bevorzugt ist ein erfindungsgemäßes Verfahren, wobei die Probenmasse für die Proben aus Fettgewebe ≤ 50 mg, bevorzugt ≤ 20 mg und weiter bevorzugt ≤ 5 mg ist.

Überraschenderweise hat sich gezeigt, dass selbst bei sehr kleinen Probevolumen aus Fettgewebe zuverlässig differenzierte Ergebnisse erzielt werden können. Dabei ist es besonders bevorzugt, dass die Probe durch Punktion als Feinnadelaspirat gewonnen wurde.

Die Bestimmung der jeweiligen Parameter aus Fettgewebe besitzt aus Sicht der Erfinder auch im Sinne der Prognose folgende Vorteile: Zum einen können nach der Bestimmung der Parameter aus Fettgewebe erfindungsgemäß unterschiedliche Risikogruppen von Individuen, die bereits an Diabetes mellitus Typ II erkrankt sind, erkannt werden ohne dass z.B. weitere Bestimmungen von Blutproben erforderlich sind. Zum anderen ermöglicht die erfindungsgemäße Bestimmung der Parameter aus Fettgewebe frühzeitiger Individuen zu identifizieren, die eine erhöhte Wahrscheinlichkeit der Ausbildung eines Diabetes mellitus Typ II besitzen als z.B. herkömmliche HbA1c-Tests. Diese HbA1c-Tests geben nur Auskunft über den Blutglukose-Spiegel der letzten vier bis 12 Wochen wieder und sind somit für eine längerfristige Prognose zur Ausbildung eines Diabetes mellitus Typ II eher weniger geeignet.

Die Messung von IL-6 im Blutplasma lässt dagegen zum Teil wenig Rückschlüsse auf metabolische Dysfunktionen zu, da der IL-6-Spiegel durch verschiedene Zustände erhöht sein kann, z. B. durch virale oder bakterielle Infektionen, intraamniotische Infektionen (Chorioamnionitis), Myxom des Herzens, Morbus Castleman, rheumatoide Arthritis, Nierenzellkarzinom, schwere Verbrennungen. Außerdem gibt es Hinweise, dass sich IL-6 aus unterschiedlichen Quellen hinsichtlich seiner Wirkung auf Zielgewebe unterscheidet. Mit einer Bestimmung des IL-6-Spiegels im Blut lässt sich nicht differenzieren, wie hoch der Anteil des aus dem Fettgewebe stammenden IL-6 ist. Außerdem ist die Halbwertszeit von IL-6 im Blut gering, so dass es zwar zur Diagnostik akuter Entzündungsvorgänge herangezogen werden kann, für die Erkennung einer chronischen inflammatorischen Reaktion aber ungeeignet ist. Die direkte Messung der mRNA im Fettgewebe als IL-6-bildendes Organ ist von solchen Faktoren unabhängig und bietet sich daher für die individuelle Risikoabschätzung im Hinblick auf mit Adipositas assoziierte Folgeerkrankungen besonders an.

Erfindungsgemäß bevorzugt ist der Proband ein Mensch, da eine differenzierte Prognose und Diagnose im Falle von T2D bei Menschen von einer ganz besonderen Bedeutung sowohl wirtschaftlicher als auch gesundheitspolitischer Natur ist.

Wie bereits oben angedeutet ist es bevorzugt, dass die Bestimmung des Genexpressionslevels auf mRNA-Ebene erfolgt. So können zuverlässige Daten mit äußerst geringen Probenmengen und mittels etablierter Methoden gewonnen werden.

Bevorzugt ist ein erfindungsgemäßes Verfahren, wobei die Eingruppierung in die Risikogruppen (in Schritt b)) unter Verwendung des multivariaten Modells der selbstorganisierenden Karten nach Kohonen erfolgt.

Zu der Methodik der selbstorganisierenden Karten wird auf den untenstehenden Methodikteil verwiesen.

Erfindungsgemäß bevorzugt ist eine Verwendung oder ein Verfahren, wobei die Einordnung in Schritt b) in eine von wenigstens 5 Gruppen erfolgt, wobei wenigstens zwei der Gruppen aus Individuen bestehen, die eine erhöhte Wahrscheinlichkeit der Ausbildung eines Diabetes mellitus Typ II besitzen und/oder wenigstens zwei der Gruppen aus Individuen bestehen, die Diabetes mellitus Typ II entwickelt haben und/oder wenigstens zwei Gruppen aus Individuen bestehen, die keinen Diabetes mellitus Typ II ausgebildet haben.

Es hat sich nämlich herausgestellt, dass mittels der Berücksichtigung des relativen Genexpressionslevels von *IL-6* es möglich ist, auch innerhalb der Gruppen der Individuen/Probanden, die eine erhöhte Wahrscheinlichkeit zur Ausbildung eines Diabetes mellitus Typ II besitzen und/oder bereits T2D ausgebildet haben und/oder kein T2D ausgebildet haben, eine Differenzierung auszubilden. Auch aus dieser Erkenntnis lassen sich therapeutische Ansätze ableiten bzw. geeignete präventive Maßnahmen.

Weiter bevorzugt ist ein erfindungsgemäßes Verfahren, wobei das Genexpressionslevel auf mRNA-Ebene relativ zu dem Genexpressionslevel eines housekeeping Gens gemessen wird. Diese Methodik eignet sich in besonderem Maße dazu, zuverlässige Ergebnisse aus geringen Probenmengen zu erhalten.

Bevorzugt ist ein erfindungsgemäßes Verfahren, wobei als mögliche Gruppen für eine Einordnung in Schritt b) die Gruppen mit der Markersituation

I) erniedrigtes relatives Genexpressionslevel für *IL-6,* stark erhöhtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 60, und

II) leicht erhöhtes relatives Genexpressionslevel für *IL-6,* erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 60,

zur Verfügung stehen.

In diesen beiden bevorzugten zur Verfügung stehenden Eingruppierungsmöglichkeiten drückt sich das zusätzliche Potential der Verwendung der Daten von *IL-6* in besonderer Form aus. Mit den jeweiligen Kombinationen der Markerwerte (die Kombination von stets drei Werten) lassen sich - neben den Eingruppierungsmöglichkeiten in weitere Gruppen -die erfindungsgemäß bevorzugten Eingruppierungsmöglichkeiten anwenden. Hierdurch ist es möglich, eine zuverlässige zusätzliche Aussage einer Unterscheidbarkeit der an T2D erkrankten Probanden/Individuen zu gewinnen.

Bevorzugt ist ein erfindungsgemäßes Verfahren, wobei es mögliche Gruppen für die Einordnung in Schritt c) die Gruppen mit der Markersituation

III) stark erhöhtes relatives Genexpressionslevel für *IL-6,* stark erhöhtes relatives Genexpressionslevel für *HMGA2* und erhöhte HbA1c-Blutwerte sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq 65$, und

IV) leicht erniedrigtes relatives Genexpressionslevel für *IL-6,* erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\leq 60$, und/oder

V) erniedrigtes relatives Genexpressionslevel für *IL-6,* erniedrigtesrelatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\geq 50$,

In den Gruppen IV und V sind nur Individuen enthalten, die die Krankheit nicht ausgebildet haben. Die Gruppe III umfasst auch Individuen, die bereits klinische Symptome eines T2D zeigen.

Hinsichtlich der Beschreibung "erhöht"/"erniedrigt" gilt im Zweifelsfall folgendes: Für die relativen Genexpressionslevel liegt ausgehend vom Mittelwert des jeweiligen Patientenkollektives folgende Situation vor:

| | |
|---|---|
| +/- 5% | unverändert |
| + 5-10 % | leicht erhöht |
| - 5-10 % | leicht erniedrigt |
| + 5-50% | erhöht |
| - 5-50 % | erniedrigt |
| +/ > 50 % | stark erhöht |
| -/ > 50 % | stark erniedrigt |

Für die Blutwerte gilt die Abstufung entsprechend, wobei hier als Bezugswert der in Tabelle 1 angegebene Wert gilt und nur der Bereich betrachtet wird, der jenseits des Normenbereichs liegt (es gibt also stets entweder nur "erhöhte" oder "erniedrigte" Werte der jeweiligen Abstufungen neben den "unveränderten" Werten.

Selbstverständlich umfasst das Patentenkollektiv, das Grundlage ist, stets auch bereits an T2D erkrankte Individuen.

Zur Aufteilung der Gruppen wird auch auf die Beispiele verwiesen.

Teil der Erfindung ist auch ein Kit für ein erfindungsgemäßes Verfahren, umfassend

a) ein Primerpaar, das an die cDNA von *HMGA2* bindet und

b) ein Primerpaar, das an die cDNA von *IL-6* bindet und bevorzugt

c) ein Primerpaar, das an die cDNA eines housekeeping Gens ausgewählt aus der Gruppe bestehend aus *HPRT,* 18S rRNA, *GAPDH, GUSB, PBGD, B2M, ABL, RPLP0 bindet.*

Mit diesem Kit ist es möglich, für das erfindungsgemäße Verfahren die Genexpressionslevel von *IL-6* und *HMGA2* festzustellen. Die genannte Primerpaarkombination eignet sich somit für eine bevorzugte Variante des erfindungsgemäßen Verfahrens.

Nachfolgend wird die Erfindung anhand von Beispielen und unter Berücksichtigung der jeweiligen Methodik näher erläutert.

Auswerteverfahren

Methodik

Ziel der angewendeten Methoden war es, neue Klassifikationen (Cluster) von Diabetikern und Nichtdiabetikern auf der Basis verschiedener Biomarker wie z. B. HMGA2, ADIPOQ, IL-6 oder PPAR-gamma zu erstellen, die diagnostisch aber auch zukünftig therapeutisch die bisherigen Klassifikationen erweitern.

Zur formalen Beschreibung der Studiendaten wurden die üblichen Methoden der deskriptiven Statistik verwendet. Für nominale Parameter wurden absolute und relative Häufigkeit angegeben, für ordinale Parameter zusätzlich der Median. Für metrische Werte wurden Mittelwert und Standardabweichung berechnet. Normalverteilungen wurden mit Hilfe des Kolmogorow-Smirnow-Tests (KS-Test) überprüft. Nicht-parametrische Korrelationen zwischen den Biomarken wurden mit Hilfe von Kendall tau-b berechnet. Für Vergleiche zwischen kategoriellen Variablen wurde der $\chi2$ Test verwendet

Zur Berechnung der a priori nicht bekannten Cluster wurden selbstorganisierende Karten (selforganizing maps (**SOM**)) verwendet. SOMs (hier Kohonen-Karten nach Teuvo Kohonen, vgl. Teuvo Kohonen: Self-Organizing Maps. Springer-Verlag, Berlin 1995, ISBN 3-540-58600-8) sind Typen von künstlichen neuronalen Netzen mit einem unüberwachten Lernverfahren mit dem Ziel eine topographische Merkmalskarte in Form von Clustern des Eingaberaums (Patientendaten) zu erreichen. Patienten innerhalb eines Clusters sollen hierbei maximal homogen und zwischen den Clustern maximal inhomogen sein. SOMs werden verwendet zum Clustering, zur Visualisierung komplexer Zusammenhänge, Vorhersage (Auswertung), Modellierung und Datenexploration. Das hier verwendete Netz besteht aus 1000 Neuronen, Korrelationen wurden automatisch kompensiert und fehlende Werte berücksichtigt. Zur Erzeugung der Cluster wurde das SOM-WARD clustering-Verfahren verwendet (2-stufiger hierarchischer Clusteralgorithmus). Farbkodierungen wurden mit Heat Maps durchgeführt. Die hierdurch erzeugten Cluster wurden deskriptiv verglichen. Zur Beschreibung der Cluster mit Hilfe von Entscheidungsbäumen bzw. Fakten und Regeln wurden verschiedene Klassifikationsalgorithmen verwendet wie C5.0, CART und Exhausted Chaid. Als Gütemaß für die verschiedenen Klassifikatoren wurden Klassifikationsgenauigkeit, Kompaktheit des Modells, (z.B. Größe eines Entscheidungsbaums) Interpretierbarkeit des Modells, Effizienz und Robustheit gegenüber Rauschen und fehlenden Werten bewertet.

Zur weiteren Validierung der Modelle und zur Berechnung der Wichtigkeit der Biomarker für die verschiedenen Klassifikationsmodelle wurden als Vorhersagemodell RBF-Netze (Radiale Basisfunktionen Netzwerke) erstellt. Durch die RBF-Netze erhält man eine geeignete Approximation der Clusterzuordnung der SOMs. Die Eingabevektoren wurden normalisiert (Subtraktion des Mittelwerts und Division durch den Bereich (x-Min)/(Max-Min); Normalisierte Werte liegen im Bereich zwischen 0 und 1). Als Aktivierungsfunktion wird die Softmax-Funktion $\sigma$ als normalisierte radiale Basisfunktion verwendet. Softmax $\sigma$ bildet einen k-dimensionalen Vektor z auf einen k-dimensionalen Vektor $\sigma(z)$ ab.

Die Netzleistung (wie "gut" ist das Netz) wurde anhand folgender Daten überprüft:

- *Modellzusammenfassung*: Ergebnisse einschließlich Fehler, Relativer Fehler oder Prozentsatz der falschen Vorhersagen.

- *Klassifikationsergebnisse:* Für jede abhängige Variable wurde eine Klassifikationstabelle angegeben.

- *ROC-Kurven:*. ROC-Kurven (Receiver Operating Characteristic curves) geben die Sensitivität und Spezifität für jeden möglichen Cutpoint der Eingangsvariablen an. Die Area under the Curve AUC ist ein Maß für die Qualität der Klassifikation. sowie

- *Kumulative Gewinndiagramme.*

Es wurden im Einzelnen folgende Methoden verwendet:

1. Selbstorganisierende neuronale Netzwerke ($\rightarrow$ Kohonen-Karten)

2. Klassifikationsalgorithmen

- Entropiebasierte Lernverfahren (C5.0)

- Exhausted Chaid und

- CART

3. Radiale Basisfunktionen (spezieller Typ von neuronalen Netzen)

4. Deskriptive und induktive Statistik

## 1) Selbstorganisierende neuronale Netzwerke

Als Selbstorganisierte Karten (selforganizing maps (**SOM**)) bezeichnet man Typen von künstlichen neuronalen Netzen mit einem unüberwachten Lernverfahren mit dem Ziel eine topologische Darstellung des Eingaberaums (hier Patientendaten) zu erreichen. Die bekanntesten SOMs sind die topologieerhaltenden Kohonen-Karten nach Teuvo Kohonen. Der Lernalgorithmus erzeugt selbständig Klassifikatoren, nach denen es die Eingabemuster in (bisher nicht bekannte) Cluster einteilt. Ziel ist es, zu erreichen, dass die Patienten innerhalb eines Clusters maximal homogen und zwischen den Clustern maximal inhomogen sind.

**Kernidee** (Topographische Merkmalskarte): "Benachbarte" Eingabevektoren (hier Patientendaten) sollten zu benachbarten Neuronen in der Karte gehören so dass die Dichte und Verteilung der Neuronen dem Wahrscheinlichkeitsmodel der Trainingsmenge entsprechen.

**Vorteile:** Nachbarschaftsbeziehungen im "unübersichtlichen" Inputraum können direkt in der Ausgabeschicht abgelesen werden.

**Anwendungen:** SOMs werden verwendet zum Clustering, zur Visualisierung komplexer Zusammenhänge, Vorhersage (Auswertung), Modellierung und Datenexploration. Die Schwerpunkte der Anwendung für die hier vorliegenden Fragestellungen sind Clustering, Visualisierung und Vorhersage.

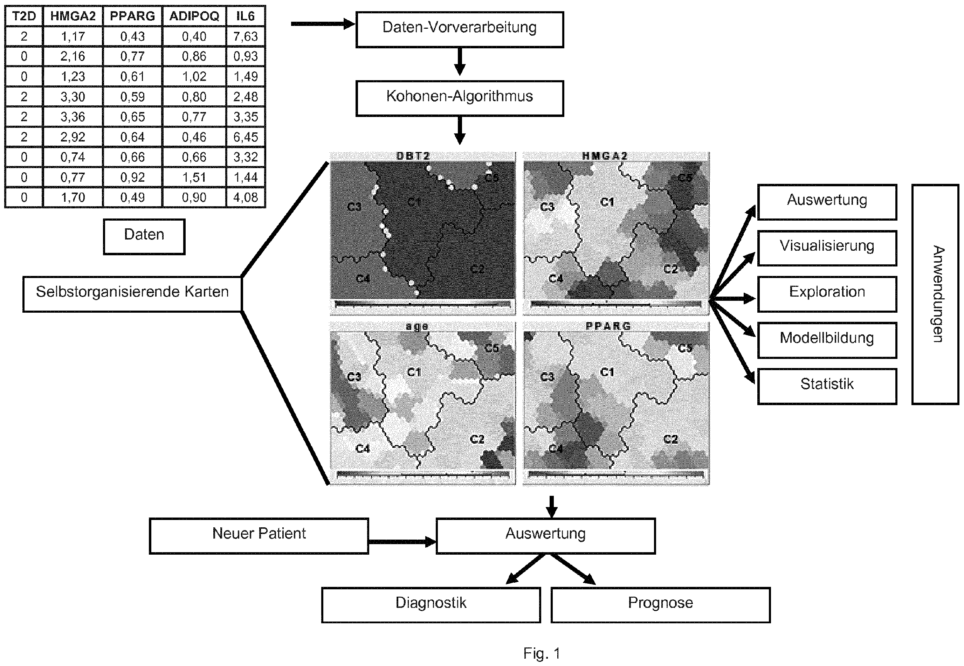

(Werkzeuge: z.B. **Self Organizing Maps in R** (R ist eine freie Programmiersprache für statistische Berechnungen und Grafiken. R ist Teil des GNU-Projekts, vgl. auch https://cran.r-project.org/web/packages/som/som.pdf und Figur 1)

## 1.1) Formale Beschreibung des Kohonen-Netzwerkmodells (Algorithmus)

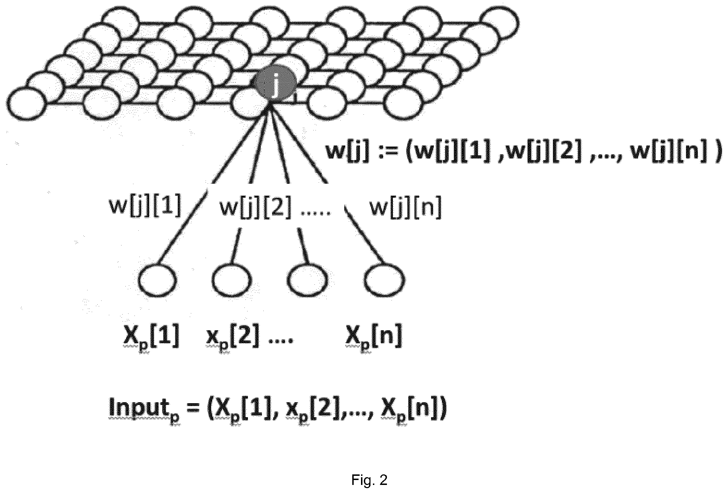

- Ein SOM besteht aus zwei Schichten von Neuronen (**Eingabeschicht** und **Ausgabeschicht**), vgl. auch Figur 2.

- Jedes Neuron der Eingabeschicht ist mit jedem Neuron der Ausgabeschicht verbunden (die Neuronen der Eingabeschicht sind vollständig mit denen der Ausgabeschicht vernetzt). Jedes Neuron der Eingabeschicht entspricht einem Parameter des Datensatzes. Die Anzahl der Eingabeneuronen ist die Dimension der Eingabeschicht. Die Ausgabeneuronen stehen durch eine Nachbarschaftsfunktion miteinander in Beziehung.

- Die Stärke der Verbindung wird durch eine Zahl (**=Gewicht) w[i][j]** repräsentiert (w[i][j] ist das Gewicht, das die Stärke der Verbindung zwischen dem i-ten Eingabeneuron und dem j-ten Ausgabeneuron angibt). Der Vektor w[j] repräsentiert alle Gewichte w[i][j] (i=1 ...n, n ist die Anzahl der für jeden Patienten erfassten Parameter) zu dem j-ten Ausgabeneuron. Eingabevektoren und Gewichtsvektoren sind normalisiert (Länge=1).

- **Initialisiere die Gewichtsvektoren.** Als Startvorgabe werden für die Gewichte beliebige durch einen Zufallsgenerator erzeugte Werte vorgegeben.

- Jeder Patient p definiert durch seine Werte einen Eingabevektor **Input$_p$ = (x$_p$[1], x$_p$[2],..., X$_p$[n])** mit den Komponenten Input$_p$[i] = x$_p$[i]. Diese Eingabevektoren werden zuerst auf 1 normalisiert.

- Für jeden Patienten p und jedes Neuron j in der Ausgabeschicht wird der euklidische Abstand

$$s_p[j] = \| \mathbf{w}[j] - \mathbf{input}_p \| = \sqrt{\sum_i \left( \mathbf{w}[j][i] - \mathbf{input}_p[i] \right)^2}$$

zwischen dem Gewichtsvektor w[j] und dem Eingabevektor Input$_p$ berechnet.

- Das Ausgabeneuron das den kleinsten Abstand zum Eingabevektor Input$_p$ hat, heißt **Gewinnerneuron ("Winner-Takes All").** Der Gewichtsvektor des Gewinnerneurons ist dem Eingabevektor am ähnlichsten d.h. hat die "maximale Erregung" unter dem Eingabevektor Input$_p$. Sollten 2 oder mehr Ausgabeneuronen den gleichen minimalen Abstand haben, so wird per Zufallsgenerator ein Neuron ausgewählt.

- Das **Gewinnerneuron** sowie seine Nachbarn erhalten den "Zuschlag", dürfen also den Input repräsentieren

- Hiermit ist eine Funktion f(input_p) definiert, die jedem Vektor input_p des Inputraumes (Musterraum, Merkmalsraum) einen Ort $\alpha$ in einer repräsentierenden Schicht (Karte) zuweist
f: input_p $\rightarrow$ f(input_p) .= arg min($\|$w[j] - input_p$\|$). Das Minimum wird über alle Gewichtsvektoren w[j] gebildet. Die Funktion arg liefert den Index des Gewinnerneurons.

- Bestimme für jeden Patienten (Eingabemuster) das Gewinnerneuron nach obiger Vorschrift.

- **Nachbarschaftsfunktion** und **Gewichtsadaption:** Im nächsten Schritt werden die Gewichte des Siegerneurons und die seiner umliegenden Neuronen adaptiert. Dabei spielt der Grad der Nachbarschaft zum Gewinnerneuron eine große Rolle. Nehmen wir an, das Gewinnerneuron hat den Index $\alpha$. Für einen Input[i] und ein Ausgabeneuron j bezeichne Dw[j][i] die Gewichtsänderung im Rahmen einer Lernregel. Diese wird wie folgt berechnet:

$$\textbf{Dw[j][i]} = \eta(t) * (\textbf{input[i]} - \textbf{w[i][j]} ) * \textbf{NbdWt}(\alpha, \textbf{j})$$

wobei

$$\eta(\textbf{t}) \textbf{ die Lernrate} (\eta(t) \text{ mit } 0 < \eta(t) < 1)$$

und NbdWt($\alpha$, j) die **Nachbarschaftsfunktion** (neighborhood weighting Funktion) bezeichnet. Eine Nachbarschaftsfunktion berechnet den Grad der Nachbarschaft eines Ausgabeneurons zum Gewinnerneuron, welcher zwischen 0 und 1 liegt. Konvergenzbeweise gegen einen statistisch beschreibbaren Gleichgewichtszustand existieren z.B. für $\eta(\textbf{t}) := \eta * \textbf{t}^{-a}$ **mit 1<a$\leq$1.**

- **Nähere Gewichtsvektoren proportional zur Nachbarschaftsfunktion zueinander an** Die Gewichtsvektoren w[j] aller Neuronen j gemäß der Nachbarschaftsfunktion NbdWt($\alpha$, j) des Neurons $\alpha$ werden aktualisiert. Das neue Gewicht $\textbf{w}_{neu}$[i][j] berechnet sich wie folgt:

-

$$\textbf{w}_{\textbf{neu}}\textbf{[j][i]} := \textbf{w[j][i]} + \textbf{Dw[j][i]}$$

- Die Nachbarschaftsfunktion kann z.B. eine der folgenden Funktionen sein :

$$NbdWt(\alpha, j) = \frac{1}{1 + \left(\dfrac{d(\alpha, j)}{s}\right)^2}$$

$$NbdWt(\alpha, j) = e^{-\left(\frac{d(\alpha, j)}{s}\right)^2}$$

mit d($\alpha$, j):= ( $\alpha$ - j); s ist ein Skalar. Die Breite s = s(t) der Nachbarschaftsfunktion und die Lernschrittweite $\eta$(t) werden im Lauf der Zeit verringert: Das GewinnerNeuron und seine Nachbarn werden gemäß der Funktion NbdWt(a, j) "aktiviert".

- Normiere Gewichtsvektoren auf Länge 1, präsentiere nächsten Datenpunkt.

    Für die verschiedenen Verfahren zur Farbkodierungen (Heat Maps) von Karte wird auf die Literatur verwiesen (z.B.
    http://citeseerx.ist.psu.edu/viewdoc/download?doi=10.1.1.100.500&rep=rep1&type =pdf, https://arxiv.org/pdf/1306.3860.pdf

oder

https://www.visualcinnamon.com/2013/07/self-organizing-maps-creating-hexagonal.html ).

**2) Klassifikationsalgorithmen**

Zur Auswertung der SOM-Modelle (Beschreibung der Klassen durch Fakten und Regeln bzw. Entscheidungsbäume) werden 3 verschiedene Klassifikationsalgorithmen verwendet: (1) Entropiebasierte Lernverfahren (C5.0), (2) Exhausted Chaid und (3) CART.

**2.1) Definition.**

Klassifikationsverfahren sind Methoden und Kriterien zur Klassifizierung von Objekten (hier Patienten) in Klassen (hier Typen und Subtypen von Gesunden, Prädiabetikern bzw. Diabetikern).

Aus einer Trainingsmenge von Beispielen mit bekannter Klassenzugehörigkeit wird mit Hilfe des Klassifikationsalgorithmus ein **Klassifikator in Form von Entscheidungsbäumen oder äquivalent in Form von Fakten und "Wenn-Dann" Regeln** generiert. Die Klassifikation grenzt sich vom Clustering (s.a. SOMs) dadurch ab, dass bei der **Klassifikation** die Klassen apriori bekannt sind, während beim **Clustering** die Klassen erst gesucht werden müssen.

**Entscheidungsbäume** dienen zur Entscheidungsfindung durch eine baumartige Struktur, die aus einem Wurzelknoten (Startknoten), Knoten, Kanten und Blättern (Endknoten) bestehen (Figur 2).

Formal ist ein Baum ein endlicher Graph mit den Eigenschaften:

1) Es gibt genau einen Knoten, in dem keine Kante endet (die **"Wurzel"**).

2) In jedem von der Wurzel verschiedenen Knoten endet genau eine Kante, die Kanten sind gerichtet

3) Jeder Knoten ist von der Wurzel auf genau einem Pfad erreichbar.

Ein **Entscheidungsbaum** ist ein Baum:

1) Jeder Knoten testet ein Attribut

2) Jeder Zweig korrespondiert zu einem Attributwert

3) Jedes Blatt (Knoten ohne ausgehende Kanten) ordnet eine Klasse zu.

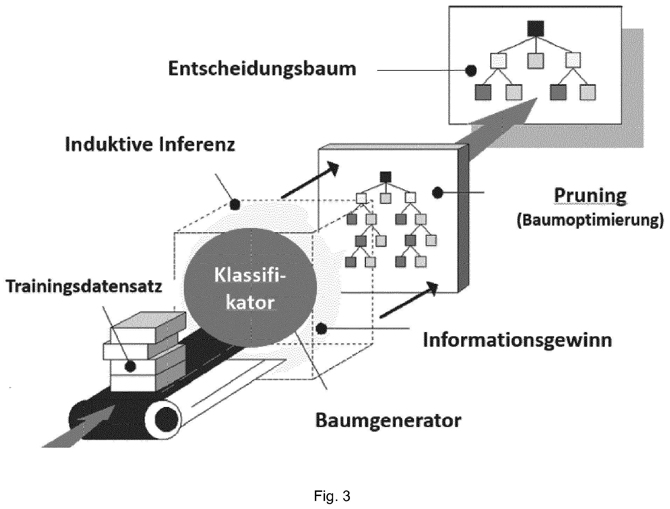

Die Figur 3 zeigt die formalen Komponenten eines Klassifikationsalgorithmus.

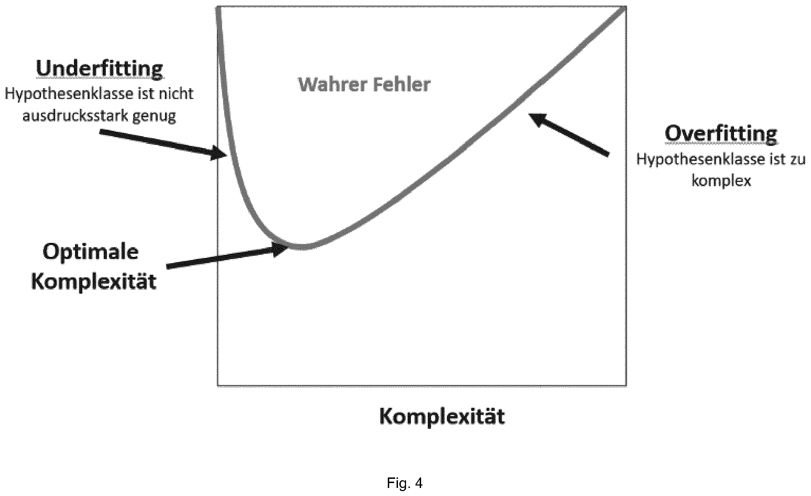

Problem: Der Klassifikator ist im ersten Schritt für die Trainingsdaten optimiert. Er kann auf der Grundgesamtheit der Daten evtl. schlechtere Ergebnisse (Underfitting oder Overfitting: Hypothesenklasse ist zu ausdrucksschwach oder zu komplex) liefern, vergleiche Figur 4.

Ein mögliches Overfitting kann durch **Pruning-oder Boosting-Verfahren** verringert werden, hierbei wählt der Fachmann die Zahl der benötigten Iterationsschritte zur Verbesserung der Gruppenbildung.

Generell teilt man die Menge der vorhandenen Beispiele in zwei Teilmengen (Train-and-Test).

- **Trainingsmenge:** zum Lernen des Klassifikators (Konstruktion des Modells)

- **Testmenge:** zum Bewerten des Klassifikators

Ist dies ist nicht anwendbar, weil die Menge der Objekte mit bekannter Klassenzugehörigkeit klein ist, so wird anstelle von Train-and-Test die sogenannte m-fache **Cross-Validierung** verwendet.

Als **Gütemaße für Klassifikatoren** nimmt man folgende Kriterien:

- Klassifikationsgenauigkeit

- Kompaktheit des Modells (z.B. Größe eines Entscheidungsbaums)

- Interpretierbarkeit des Modells.

- Effizienz

- Robustheit gegenüber Rauschen und fehlenden Werten

## 2.2) Konstruktion von Entscheidungsbäumen

(Vergleiche auch

- Quinlan, J. Ross (1986): Induction of decision trees. In: Machine Learning 1 (1), S. 81-106.
- Quinlan, J. Ross (1993): C4.5. Programs for machine learning. In: J. Ross Quinlan. San Mateo, Calif.: Morgan Kaufmann (The Morgan Kaufmann series in machine learning).
- Quinlan, J. Ross (1996): Improved use of continuous attributes in C4. 5. Journal of artificial intelligence research 4, S. 77-90.)

### Basis-Algorithmus

Schleife:

1. Wähle das "Beste" Entscheidungsattribut A für den nächsten Knoten
2. Für jeden Wert von A erzeuge einen neuen Abkömmlingsknoten
3. Ordne die Trainingsdaten den Abkömmlingsknoten zu
4. Wenn die Trainingsdaten fehlerfrei klassifiziert werden, dann STOPPE. Sonst iteriere über Abkömmlingsknoten ($\rightarrow$ 1.).

**Bezeichnungen:**

Trainingsdatensatz T,

Anzahl der Trainingsdaten |T|,

Klassen $C_i$: Der Datensatz aller Trainingsdaten in der Klasse $C_i$. |$C_i$| list die Anzahl der Elemente in Klasse $C_i$. Es gilt: $\sum |C_i| = |T|$ (i=1,..k).

Attribut $A = \{a_1, a_2, ... , a_m\}$. Das Attribut A unterteilt den Datensatz T in m Teilsätze $T_1, T_2, ... ,T_m$. |$T_i$| ist die Anzahl der Teilmenge $T_i$.

Gegeben Trainingsdatensatz T und Attribute A;

Output: Informationsgewinn(T, A) von Attribute A für den Trainingsdatensatz T

### 3) Algorithmus (Berechnung des Informationsgewinns mit Hilfe der Entropie am Beispiel von ID3)

- Die empirische *Entropie* **für eine Menge** *T* von Trainingsobjekten mit den Klassen $C_i$ (i=1,...,k) ist definiert als

$$entropie(T) = \sum_{i=1}^{k} p_i \cdot \log p_i$$

mit $p_i := |C_i|/|T|$

- Das Attribut A habe die Partitionierung $T_1, T_2, ..., T_m$ erzeugt. Die empirisch bedingte *Entropie G(T,A)* **für eine Menge** *T* **und ein Attribut A** ist definiert als

$$G(T, A) = \sum_{i=1}^{m} \frac{|T_i|}{|T|} \cdot entropie(T_i)$$

- Der *Informationsgewinn* Gain(T, A) durch das Attribut A in Bezug auf T ist definiert als

$$Gain(T, A) = entropie(T) - G(T, A) = entropie(T) - \sum_{i=1}^{m} \frac{|T_i|}{|T|} \cdot entropie(T_i)$$

**Input:** Trainingsdatensatz T mit den Klassen $C_i$ (i=1, ...,k), Attribute A und Schwellenwert $\varepsilon$ ;

**Output:** Entscheidungsbaum E;

**Algorithmus:**

1. Erstellen eines Knotens K;

2. Falls alle Beispieldaten in T eine identische Klasse $C_j$ haben oder die Anzahl der Daten kleiner als Schwellenwert $\varepsilon$ ist, dann wird der einzige Knoten mit Klasse $C_j$ zum Knoten K als Blatt zurück;

3. Falls $A = \emptyset$, dann wird der einzige Knoten mit der häufigsten Klasse in T zum Knoten K als Blatt zugeordnet;

4. Berechnung des Informationsgewinns Gain von A in T und Bestimmung des besten Attributs $A_g$ mit maximalem Informationsgewinn anhand

$$Gain(T, A) = entropie(T) - G(T, A) = entropie(T) - \sum_{i=1}^{m} \frac{|T_i|}{|T|} \cdot entropie(T_i)$$

5. Bezeichnen den Knoten K mit $A_g$

6. Berechne für alle Attributwerte $A_{gj}$ von $A_g$ den Teildatensatz $T_{gi}$ aller Beispiele aus dem Trainingsdatensatz mit $A_{gj}$

7. Ist $T_{gi} = \emptyset$, dann wird ein Blatt mit der häufigsten Klasse zum Knoten K angefügt andernfalls

8. Rekursion des Zweiges von $A_g$.

**Bemerkung:**

Für eine Zufallsereignis y, welches mit Wahrscheinlichkeit P(y) aufritt, gilt:
**Informationsgehalt:** $h(y) \equiv -\log_2(P(y))$

Die Entropie ist der mittlere Betrag des Informationsgehalts der Zufallsvariable y

$$H[y] \equiv \sum y\, P(y) h(y) = -\sum y\, P(y) \log 2(P(y))$$

**Verwendete Klassifikationsalgorithmen**

**ID3**

- Diskrete Attribute, keine fehlenden Attribute

- Information Gain als Qualitätsmaß.

**C4.5/C5.0**

- Erweiterung von ID3.

- Information GainRatio als Qualitätsmaß. Fehlenden Attribute

- Numerische und reellwertige Attribute

- Pruning des Entscheidungsbaumes

Die Information GainRatio ist definiert als

$$GainRatio\,(T,A) := \frac{\text{Gain(T,A)}}{\text{SplitInformation(T,A)}}$$

$$SplitInformation(T,A) = -\sum_{i=1}^{m} \frac{|T_i|}{|T|} \cdot \log_2(\frac{|T_i|}{|T|})$$

**CART (Classification And Regression Trees)**

**Vergleiche auch** Hastie, T., Tibshirani, R., Friedman, J. H. (2001). The elements of statistical learning: Data mining, inference, and prediction. New York: Springer Verlag.

Verfahren analog zu ID3 bzw. C5.0. Das Informationsmaß wird durch den **Gini-Index** definiert. Der Gini Index wird minimiert (anstatt den Gini Gain zu maximieren).

Gini gain

$$Gini(T,A) = \sum_{i=1}^{k} \frac{T_i}{T} \cdot Gini(T_i)$$

$$Gini(T) = 1 - \sum_{i=1}^{k} p_i 2$$
Mit

$P_i$ ist die relative Häufigkeit der Klasse $C_i$ in T

*CHAID(Chi-squareAutomatic Interaction Detectors)*

Vergleiche auch Sonquist, J.A. and Morgan, J.N. (1964): The Detection of Interaction Effects. Survey Research Center, Institute for Social Research, University of Michigan, Ann Arbor.

**CHAID** ist ein weiterer Algorithmus zur Konstruktion von Entscheidungsbäumen. Die Unterschiede zu C5.0 oder CART bestehen darin, dass zur Wahl der Attribute beim CHAID-Algorithmus der Chi-Quadrat-Unabhängigkeitstest verwendet wird und dass der CHAID-Algorithmus das Wachsen des Baumes stoppt, bevor der Baum zu groß geworden ist. Der Baum wird also nicht beliebig wachsen gelassen, um ihn danach mit einer Pruning-Methode wieder zu kürzen.

**4) Radiale Basisfunktionen Netze (RBF-Netze)**

Vergleiche auch Zell, A. : Simulation neuronaler Netze. Oldenbourg 1994

Zur weiteren Validierung der Modelle und zur Berechnung der Wichtigkeit der Biomarker für die verschiedenen Klassifikationsmodelle werden RBF-Netze verwendet. RBF-Netze erstellen Vorhersagemodelle. Sie eignen sich besonders für die Approximation von Funktionen.

Das RBF-Netz besteht aus einer Inputschicht mit n Neuronen, einer Hidden Schicht mit k Neuronen und einer Output-Schicht mit m Neuronen. Ein n-dimensionales Muster wird hierdurch in einen m-dimensionalen Output-Raum abgebildet.

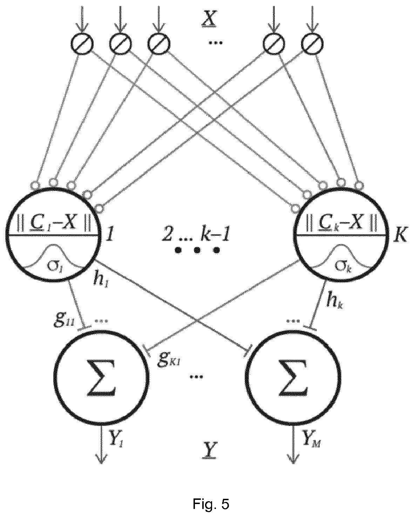

Die Inputschicht ist eine reine Weiterleitung. Jedes Neuron verteilt seinen Wert an alle verdeckten Neuronen. In der Hidden Schicht wird in jedem Neuron der Abstand zwischen der Eingabe und dem Zentrum c mit Hilfe einer euklidischen Norm gebildet. Als Netzeingabe- und Aktivierungsfunktion werden radiale Basisfunktionen verwendet (vergleiche Figur 5).

Die Aktivierungsfunktion jedes versteckten Neurons ist eine sogenannte radiale Funktion,

d.h. eine monoton fallende Funktion

$$f : IR_0^+ \rightarrow [0, 1] \text{ mit } f(0) = 1 \text{ und } \lim_{x \to \infty} f(x) = 0 \text{m}$$

Die Eingabevektoren werden **normalisiert** (Subtraktion des Mittelwerts und Division durch den Bereich (x-Min)/(Max-Min); Normalisierte Werte liegen im Bereich zwischen 0 und 1). Als Aktivierungsfunktion werden die **Softmax-Funktion** $\sigma$ als normalisierte radiale Basisfunktion verwendet. Softmax $\sigma$ bildet einen k-dimensionalen Vektor z auf einen k-dimensionalen Vektor $\sigma(z)$ ab.

$$\sigma(\mathbf{z})_j = \frac{e^{z_j}}{\sum_{k=1}^{K} e^{z_k}}, \quad j=1, \ldots, k$$

Die Anzahl der Neuronen in der verdeckten Schicht wird durch das **"Bayesian Information Criterion"** (Vergleiche Schwarz, Gideon E. (1978), "Estimating the dimension of a model", Annals of Statistics, 6 (2): 461-464, MR 468014, doi:10.1214/aos/1176344136) (BIC) bestimmt. Die beste Anzahl an verborgenen Einheiten ist diejenige, die auf der Basis der Trainingsdaten den kleinsten BIC ergibt.

Für die Ausgabeschicht benutzten wir als Aktivierungsfunktion die Identitätsfunktion. Die Ausgabeeinheiten sind also einfach gewichtete Summen der verborgenen Einheiten. Die Ausgabe des Netzwerkes ist daher eine Linearkombination der Radialen Basisfunktionen der Eingaben und der Gewichte.

### Netzleistung

Die Netzleistung überprüft, wie "gut" das Netz ist. Hierzu werden eine Reihe von Ergebnissen bereitgestellt.

- Modellzusammenfassung.
  Ergebnisse einschließlich Fehler, Relativer Fehler oder Prozentsatz der falschen Vorhersagen und Trainingszeit.

- Klassifikationsergebnisse.
  Für jede kategoriale abhängige Variable wird eine Klassifikationstabelle angegeben.

- ROC-Kurven.
  Die ROC-Kurven (Receiver Operating Characteristic curves) geben die Sensitivität und Spezifität für jeden möglichen Cutpoint der Eingangsvariablen an. Die Area under the Curve AUC ist ein Maß für die Qualität der Klassifikation.

- Kumulative Gewinndiagramme.

### Beispiele

Die nachfolgenden Beispiele beruhen auf zwei unterschiedlichen Patientenkollektiven. Das Patentenkollektiv 1 wurde als Basis für die nachfolgenden Beispiele 1 und 4 hinzugezogen. Es zeichnet sich durch eine Zahl von **15** Individuen, bei denen bei **6** ein T2D vorlag und das Patentenkollektiv 2, das die Basis für die Beispiele 2, 3 und 5 darstellt, bestand aus **61** Probanden, von denen bei **26** ein T2D vorlag.

Als Kalibrator für die relativen Genexpressionswerte diente eine Probe mit folgenden $\Delta C_T$:

| Patientenkollektiv 1: | *HMGA2*: | 6,358 |
|---|---|---|
| | *IL6:* | 7,067 |

(fortgesetzt)

| Patientenkollektiv 2: | HMGA2: | 6,513 |
| | IL6: | 6,446 |

Die $\Delta C_T$-Werte sind dabei der $C_T$-Wert des Zielgens minus dem $C_T$-Wert der endogenen Kontrolle (housekeeping Gen), wobei der $C_T$-Wert jeweils der Wert ist, bei dem in der Amplifikation erstmalig das Signal für die jeweilige cDNA den Schwellwert überscheitet.

Für die Mittelwerte der relativen Genexpression im Patientenkollektiv 2 ergaben sich folgende Mittelwerte:

| HMGA2: | 1,663117 |
| IL-6: | 3,7405 |

### Beispiel 1

### Nachweis der Expression von *IL-6* in Fettgewebspunktaten, gewonnen durch Feinnadel-Aspiration des subkutanen abdominalen Fettgewebes

### Material und Methoden

### Feinnadel-Aspiration

Feinnadel-Aspirate wurden durch Punktion von subkutanem abdominalem Fettgewebe (hWAT) mittels 20 ml Spritze und einer Einmal-Injektions-Kanüle (Durchmesser 0,90 × 40 mm) gewonnen. Nach Desinfektion der Einstichstelle wurde die Kanüle in das Unterhaut-Fettgewebe eingeführt. Mit der Spritze wurde ein Unterdruck erzeugt und die Kanüle im Gewebe fächerförmig vor und zurück bewegt, um so Zellen des Fettgewebes zu aspirieren. Direkt nach der Punktion wurden die Proben in 1 ml QIAzol Lysis Reagenz (QIAGEN, Hilden, Deutschland) aufgenommen und die Kanüle mehrmals mit dem QIAzol gespült. Anschließend wurden die Proben bei -80 °C eingefroren.

### RNA-Isolierung

Die Isolierung der Gesamt-RNA erfolgte mittels RNeasy Lipid Tissue Mini Kit (QIAGEN, Hilden, Deutschland) in einem QIAcube (QIAGEN, Hilden, Deutschland) nach Herstellerangaben. Die Feinnadel-Aspirate (5 mg) in 1 ml QIAzol Lysis Reagenz wurden in einem Tissue Lyser II (QIAGEN, Hilden, Deutschland) homogenisiert und anschließend wurde das Homogenat bei Raumtemperatur für 5 min inkubiert. Es folgte die Zugabe von 200 $\mu$l Chloroform, welches mittels kräftigem Schütteln per Hand für 15 sec mit der Probe vermischt wurde. Die Probe wurde erneut für 2 min bei Raumtemperatur inkubiert und mit 12.000 x g zentrifugiert für 15 min bei 4 °C. Anschließend wurde die obere wässrige Phase in ein neues 2 ml Cup transferiert und die Gesamt-RNA wurde über eine Qiagen RNeasy Mini Spin Säule (QIAGEN, Hilden, Deutschland) in einem QIAcube nach Herstellerangaben isoliert.

### cDNA-Synthese

Für die cDNA-Synthese wurde ≤ 250 ng RNA mittels 200 U M-MLV Reverse Transkriptase, RNase Out (Thermo Fisher Scientific, Darmstadt, Germany) und 150 ng Random-Primer (Thermo Fisher Scientific, Darmstadt, Germany) nach Herstellerangaben in cDNA umgeschrieben. Die RNA wurde bei 65 °C für 5 min denaturiert und anschließend mindestens 1 min auf Eis gelagert. Nach der Zugabe des Enzyms wurde für das Annealing der Random-Primer an die RNA der Mix für 10 min bei 25 °C inkubiert. Die anschließende Reverse Transkription wurde bei 37 °C für 50 min durchgeführt, gefolgt von einer 15 min Inaktivierung der Reversen Transkriptase bei 70 °C.

### Prä-Amplifizierung der cDNA

5 $\mu$l cDNA wurde mittels RealTime ready cDNA Preamp Mastermix (Roche, Mannheim, Germany) unter Verwendung von *IL-6-, HMGA2-* und *HPRT-* (Hypoxanthin-Phosphoribosyltransferase 1) spezifischen Primern nach Herstellerangaben präamplifiziert. Die genspezifischen Primer binden dabei in geeigneter Weise an die jeweilige cDNA, so dass die erzeugten Amplikons die Bindestellen der Primer der in der quantitativen Real-Time PCR verwendeten genspezifischen Assays enthalten. Die Prä-Amplifikation der cDNA erfolgte nach folgendem Temperaturprofil: 95 °C für 1 min gefolgt von 14 Zyklen bei 95 °C für 15 sec und bei 60 °C für 4 min.

### Quantitative Real-Time PCR (qRT-PCR)

Die relative Quantifizierung der Genexpression wurde mittels Real-Time PCR auf dem Applied Biosystems 7300 Real-Time PCR System durchgeführt. Kommerziell erhältliche Genexpressions-Assays (Life Technologies, Carlsbad, CA, USA) wurden zur Quantifizierung der mRNA von *IL-6* (Assay-ID Hs00985639_m1) und *HMGA2* (Assay-ID Hs00171569_m1) verwendet. Als endogene Kontrolle wurde, wie von Klemke *et al.* (Klemke M, Meyer A, Hashemi Nezhad M, Beige G, Bartnitzke S, Bullerdiek J (2010) Loss of let-7 binding sites resulting from truncations of the 3' untranslated region of HMGA2 mRNA in uterine leiomyomas. Cancer Genet Cytogenet 196:119-123) beschrieben, *HPRT* genutzt. Alle gemessenen Proben wurden dreifach bestimmt. Die Quantifizierung der Genexpression wurde in 96-Well Platten mit der zu untersuchenden präamplifizierten cDNA, des jeweiligen genspezifischen Assays und des FastStart Universal Probe Master (Rox) (Roche, Mannheim, Germany) durchgeführt. Das Temperaturprofil der Real-Time PCR folgte den Herstellerangaben: Bei 95 °C erfolgt eine Denaturierung des Templates für 10 min. Anschließend folgte die Amplifikation in 50 Zyklen beginnend mit der Denaturierung für 15 sec bei 95 °C und der Kombination aus Annealing/Elongation für 60 sec bei 60 °C. Die erhaltenen Daten wurden mittels komparativer Delta-Ct-Methode ($\Delta\Delta$CT-Methode) ausgewertet. [(Livak KJ, Schmittgen TD (2001) Analysis of relative gene expression data using real-time quantitative PCR and the 2 -$\Delta\Delta$C(T) Method. Methods 25: 402-408)]

### Ergebnisse

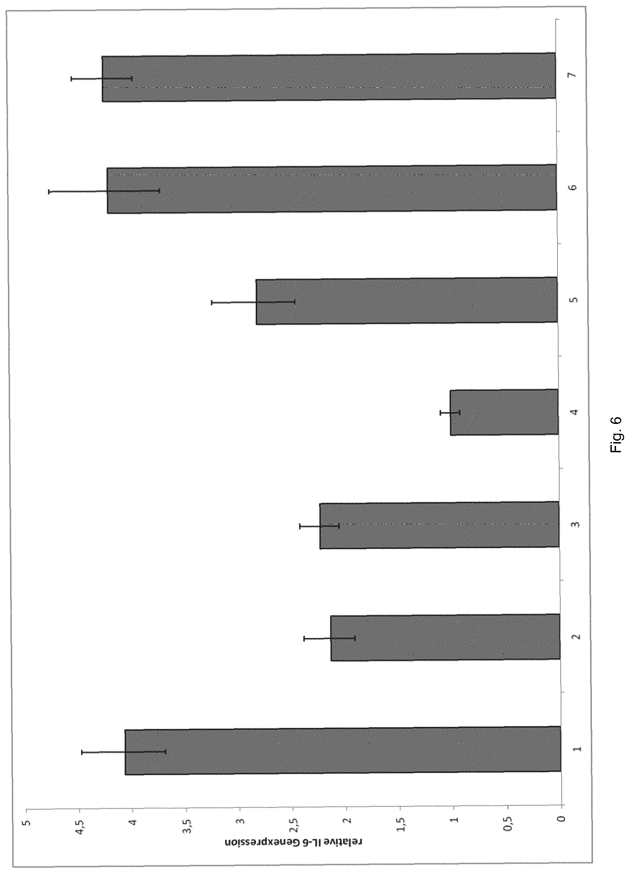

Die Genexpression von *IL-6* konnte mittels qRT-PCR in sieben Proben von humanen Patienten gemessen werden (Fig. 6). Die Ergebnisse zeigen eindeutig, dass IL-6-mRNAs zuverlässig aus Feinnadel-Aspiraten mit selbst sehr geringen Mengen an Fettgewebszellen quantifizierbar sind. Zudem reicht die Menge der isolierten RNA aus den Feinnadel-Aspiraten aus, um die Expression von weiteren Genen nach einer Prä-Amplifikation zu bestimmen.

Die Fig. 6 stellt die relative Genexpression von *IL-6* in sieben Proben dar. Sie zeigt, dass eine zuverlässige Quantifizierung der *IL-6*-Expression selbst aus sehr geringen Mengen von RNA (Konzentration $\leq$ 25 ng/$\mu$l), die durch Feinnadel-Aspiration gewonnen wurde, gelingt. Zudem zeigt Fig. 6, dass die Expression der Gene zwischen den einzelnen Proben variiert und somit interindividuelle Unterschiede detektierbar sind.

### Beispiel 2

### Die Expression von *IL-6* in Fettgewebsproben von normalgewichtigen und übergewichtigen Patienten

### Material und Methoden

### Gewebeproben

Die humanen subkutanen abdominalen Fettgewebe wurden während Operationen entnommen und nach der Operation in flüssigem Stickstoff gelagert. Anschließend wurden die Proben bei -80 °C eingefroren. Für alle verwendeten humanen Fettgewebsproben wurden die Forderungen der Deklaration von Helsinki erfüllt. Eine schriftliche Einverständniserklärung für die Verwendung der Gewebeproben wurde von den Patienten (n = 61) abgegeben.

### RNA-Isolierung

Die Isolierung der Gesamt-RNA erfolgte mittels RNeasy Lipid Tissue Mini Kit (QIAGEN, Hilden, Deutschland) in einem QIAcube (QIAGEN, Hilden, Deutschland) nach Herstellerangaben. Die Fettgewebsproben (50-100 mg) in 1 ml QIAzol Lysis Reagenz wurden in einem Tissue Lyser II (QIAGEN, Hilden, Deutschland) homogenisiert und anschließend wurde das Homogenat bei Raumtemperatur für 5 min inkubiert. Es folgte die Zugabe von 200 $\mu$l Chloroform, welches mittels kräftigem Schütteln per Hand für 15 sec mit der Probe vermischt wurde. Die Probe wurde erneut für 2 min bei Raumtemperatur inkubiert und mit 12.000 x g zentrifugiert für 15 min bei 4 °C. Anschließend wurde die obere wässrige Phase in ein neues 2 ml Cup transferiert und die Gesamt-RNA wurde über eine Qiagen RNeasy Mini Spin Säule (QIAGEN, Hilden, Deutschland) in einem QIAcube nach Herstellerangaben isoliert. Die RNA-Konzentration wurde mittels eines Photometers bestimmt und die Proben wurden anschließend bei -80 °C gelagert.

### cDNA-Synthese

Für die cDNA-Synthese wurde 250 ng RNA mittels 200 U M-MLV Reverse Transkriptase, RNase Out (Thermo Fisher Scientific, Darmstadt, Germany) und 150 ng Random-Primer (Thermo Fisher Scientific, Darmstadt, Germany) nach Herstellerangaben in cDNA umgeschrieben. Die RNA wurde bei 65 °C für 5 min denaturiert und anschließend mindestens 1 min auf Eis gelagert. Nach der Zugabe des Enzyms wurde für das Annealing der Random-Primer an die RNA der Mix für 10 min bei 25 °C inkubiert. Die anschließende Reverse Transkription wurde bei 37 °C für 50 min durchgeführt, gefolgt von einer 15 min Inaktivierung der Reversen Transkriptase bei 70 °C.

**Quantitative Real-Time PCR** (qRT-PCR)

Die Durchführung der relativen quantitativen Real-Time PCR erfolgte wie in Beispiel 1 beschrieben.

**Ergebnis**

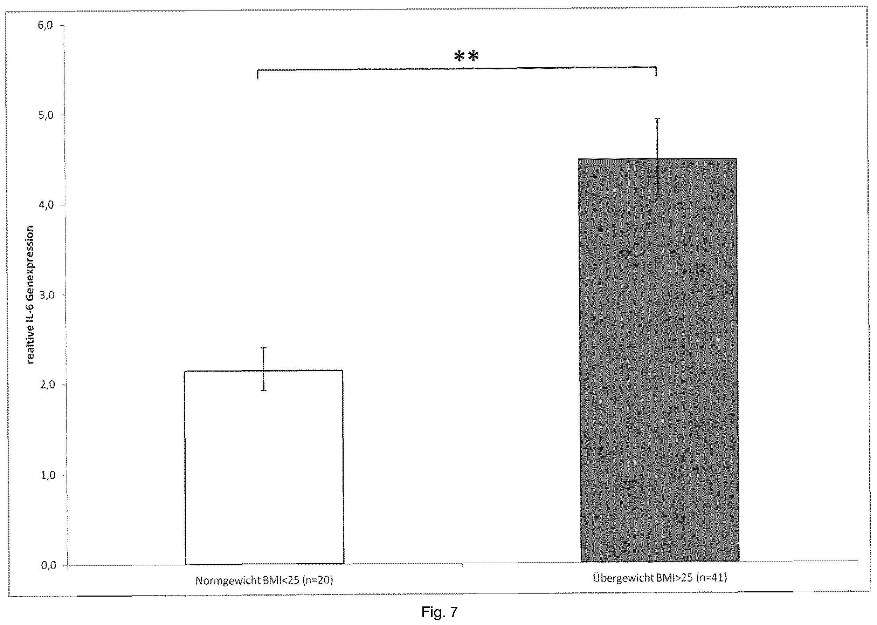

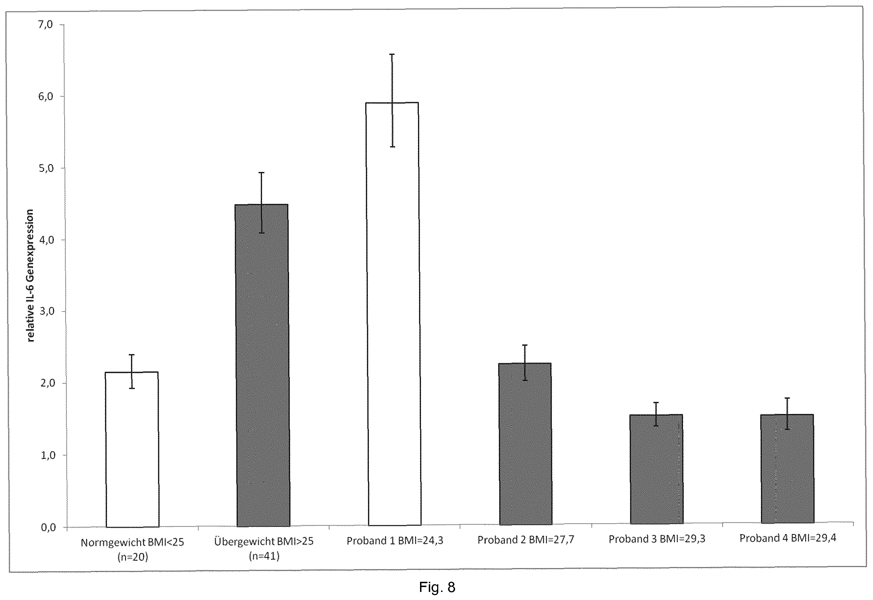

Die in der Literatur beschriebenen Unterschiede des IL-6-Levels im Blutplasma von normalgewichtigen und übergewichtigen Patienten können auch auf Ebene der Genexpression von *IL-6* im Fettgewebe nachgewiesen werden (Fig. 7). Die Fig. 7 zeigt den hoch signifikanten Unterschied ($p < 0,01$) der relativen Genexpression von *IL-6* im Fettgewebe von 20 normalgewichtigen und 41 übergewichtigen Patienten. Die *IL-6* Expression beträgt in der Gruppe mit dem Normalgewicht (n=20) 2,142 und in der Gruppe der Übergewichtigen (n=41) 4,473. Es gibt aber hiervon auch Ausnahmen, wie in Fig. 8 dargestellt, bei denen auch normalgewichtige Patienten eine stark erhöhte *IL-6* Expression und übergewichtige Patienten eine niedrige *IL-6* Expression aufweisen. Dies lässt sich dadurch erklären, dass eine subklinische Entzündungsreaktion im Fettgewebe schon vorliegen kann, diese aber noch nicht im Blut detektiert werden kann, weil der IL-6-Wert im Blut so niedrig ist, dass dieser als klinisch unauffällig betrachtet wird. Die Fig. 8 zeigt die relative Genexpression von IL-6 in Fettgewebsproben von vier Probanden im Vergleich zur Genexpression der normal- und übergewichtigen Gruppe (2,142 vs. 4,473). Proband 1 hat einen BMI von 24,3 und eine *IL-6* Expression von 5,877. Die Probanden 2 bis 4 sind alle übergewichtig und haben einen BMI von 27,7; 29,3 bzw. 29,4 und weisen eine *IL-6* Expression von 2,229, 1,507 bzw. 1,492 auf.

**Beispiel 3**

**Die Korrelation zwischen *IL-6* und dem BMI ist abhängig von *HMGA2***

**Material und Methoden**

**Gewebeproben**

Die humanen subkutanen abdominalen Fettgewebe wurden während Operationen entnommen und nach der Operation in flüssigem Stickstoff gelagert. Für alle verwendeten humanen Fettgewebsproben wurden die Forderungen der Deklaration von Helsinki erfüllt. Eine schriftliche Einverständniserklärung für die Verwendung der Gewebeproben wurde von den Patienten (n = 61) gegeben.

**RNA-Isolierung**

Die Isolierung der Gesamt-RNA erfolgte mittels RNeasy Lipid Tissue Mini Kit (QIAGEN, Hilden, Deutschland) wie unter Beispiel 2 beschrieben.

**cDNA-Synthese**

Die cDNA-Synthese erfolgte ebenfalls wie unter Beispiel 2 beschrieben.

**Quantitative Real-Time PCR** (qRT-PCR)

Die quantitative Real-Time PCR erfolgte wie unter Beispiel 1 beschrieben.

**Ergebnisse**

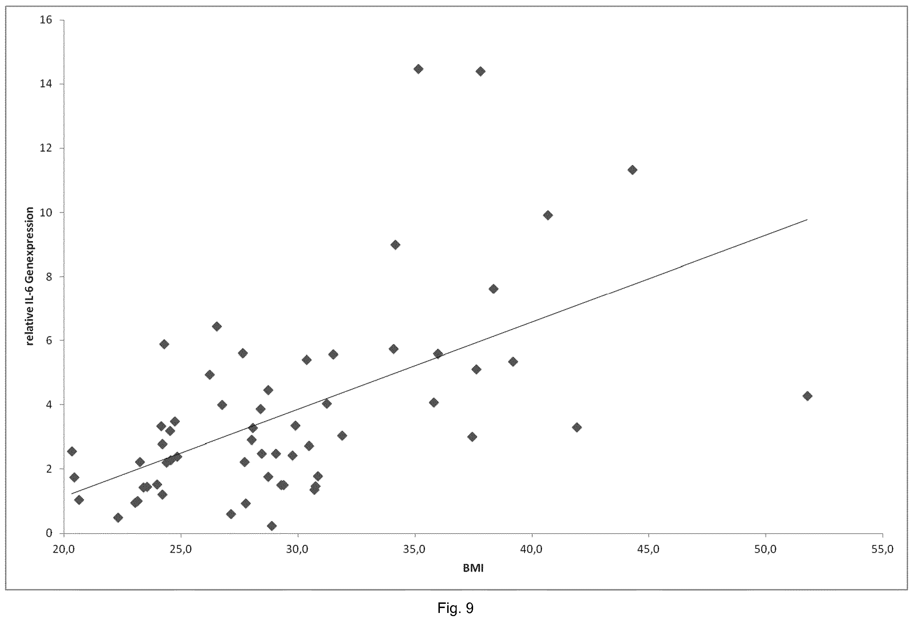

Aus Blutuntersuchungen war bereits bekannt, dass die Konzentration sowohl des C-reaktiven Proteins (CRP) als auch das IL-6-Level im Serum mit dem Body Mass Index (BMI) korreliert. Höhere BMIs gehen mit erhöhten CRP- und IL-6-Werten im Serum einher. Dieser Zusammenhang konnte auch auf der Ebene der *IL-6*-Genexpression im Fettgewebe nachvollzogen werden. Fig. 9 zeigt die an n = 60 Probanden ermittelte relative *IL-6*-Expression im Fettgewebe in Abhängigkeit vom BMI. In der Tendenz zeigte sich in Übereinstimmung mit der aus Messungen im Blut gewonnenen Erkenntnis, dass ein höherer BMI i. d. R. von einer gesteigerten *IL-6*-Expression im subkutanen Fettgewebe begleitet wird. Somit konnte der Zusammenhang zwischen BMI und *IL-6*-Expression grundsätzlich auch im Fettgewebe nachgewiesen werden, mit einem Korrelationskoeffizienten von 0,565 besteht hier jedoch eine eher mittelstarke Korrelation. Demgemäß wurden trotz der klar erkennbaren Tendenz auch Ausnahmen gefunden, d. h. auch bei normalem oder mäßig erhöhtem BMI kann in Einzelfällen eine überdurchschnittliche *IL-6*-Expression festgestellt werden, wobei dieser Effekt allerdings schwach ausgeprägt war. Häufiger und ausgeprägter trat dagegen der umgekehrte Fall auf, dass trotz eines zum Teil massiv erhöhten BMI keine Steigerung der *IL-6*-Expression im Fettgewebe nachweisbar war (Fig. 9). Für diese Beobachtung fehlte ein plausibler Erklärungsansatz bislang.

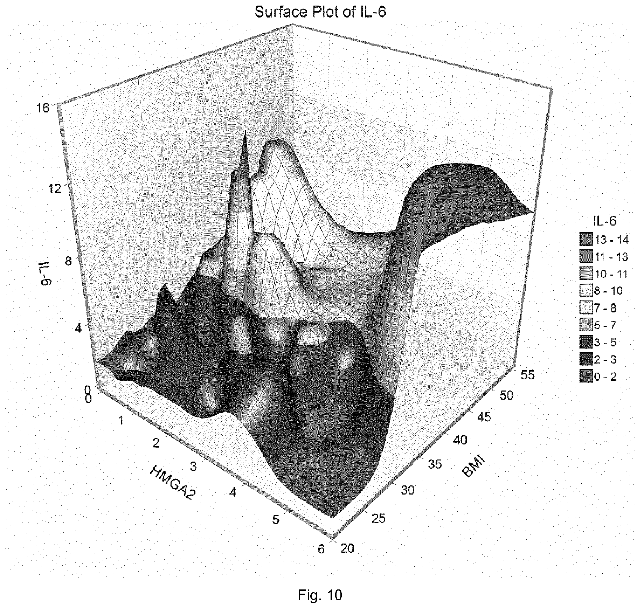

In die weiteren Auswertungen wurde als weiterer Parameter außerdem die Expression des *HMGA2*-Gens einbezogen. Die Fig. 10 zeigt die drei Parameter *IL-6*- sowie *HMGA2*-Expression und BMI in Relation zueinander. Überraschenderweise zeigte sich hierbei deutlich, dass die Korrelation zwischen *IL-6* und BMI von der Expression des *HMGA2*-Gens abhängt. Bei hohem BMI ist nur dann eine starke *IL-6*-Expression zu erwarten, wenn *HMGA2* entweder nur schwach oder aber besonders stark exprimiert wird. Liegt die *HMGA2*-Expression dagegen im mittleren Bereich, dann wird *IL-6* selbst bei extrem hohem BMI allenfalls moderat erhöht exprimiert (Fig. 10). Die Erkenntnis, dass *HMGA2* im Fettgewebe einen Einfluss auf die Korrelation zwischen IL-6 und BMI ausübt, ist völlig neu. Nie zuvor wurde beschrieben oder auch nur spekuliert, dass eine Abhängigkeit des IL-6-BMI-Zusammenhanges von *HMGA2* besteht. Daher können durchschnittliche *IL-6*-Expressionen bei sehr hohem BMI, die zuvor lediglich als statistische Ausreißer angesehen worden wären, erstmals auf eine mittlere Expression des *HMGA2*-Gens zurückgeführt werden. Nur wenn *HMGA2* entweder sehr schwach oder sehr stark exprimiert wird, gilt die beschriebene Erhöhung der *IL-6*-Expression im Fettgewebe bei Übergewicht und Adipositas. Wenn erhöhte IL-6-Level bei Adipositas das Risiko einer Typ-2-Diabeteserkrankung zusätzlich steigern (Möhlig M, Boeing H, Spranger J, Osterhoff M, Kroke A, Fisher E, Bergmann MM, Ristow M, Hoffmann K, Pfeiffer AF. 2004. Body mass index and C-174G interleukin-6 promoter polymorphism interact in predicting type 2 diabetes. J Clin Endocrinol Metab. 89: 1885-1890), dann ließe sich aus der dargelegten, neugewonnen Erkenntnis ableiten, dass adipöse Personen ein weniger stark erhöhtes Diabetesrisiko haben, wenn sie aufgrund einer durchschnittlichen *HMGA2*-Expression nicht zur Gruppe mit hohen *IL-6*-Werten zählen.

## Beispiel 4

**Unterschiedliche Genexpression von *IL-6* in Fettgewebspunktaten von Typ-2-Diabetikern**

**Material und Methoden**

**Probenvorbereitung**

Die Probengewinnung erfolgte mittels Feinnadel-Aspiration wie in Beispiel 1 beschrieben. Auch die nachfolgenden Schritte der Probenaufbereitung, also RNA-Isolierung, cDNA-Synthese, Prä-Amplifizierung der cDNA und die quantitative Real-Time-PCR erfolgten wie in Beispiel 1 beschrieben.

**Ergebnis**

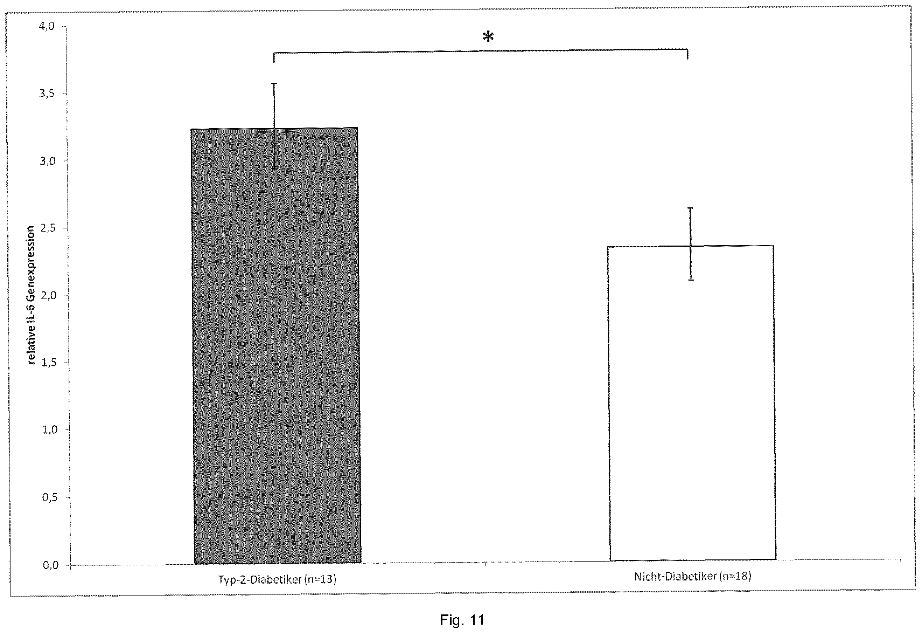

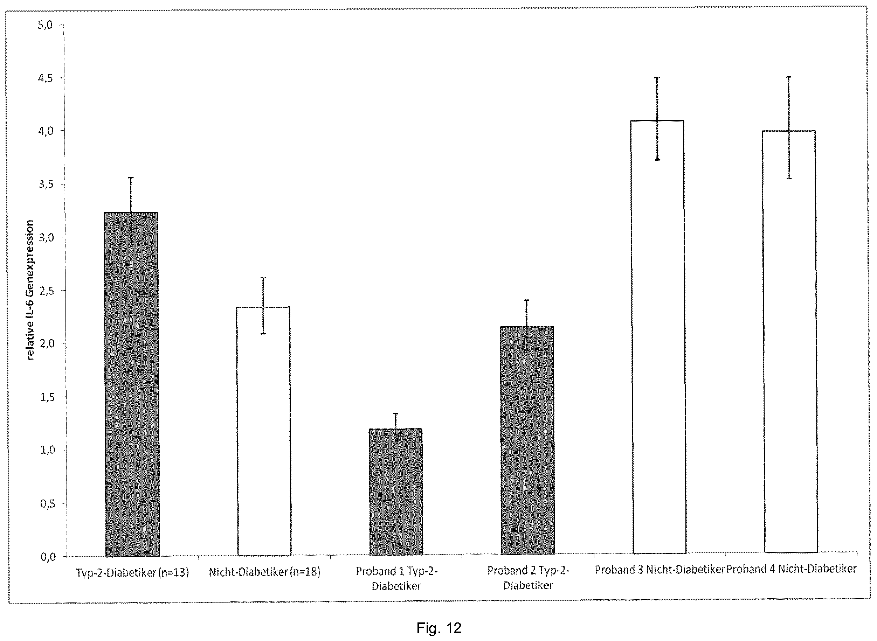

Die Fig. 11 zeigt den signifikanten Unterschied ($p < 0,05$) der relativen Genexpression von *IL-6* in Fettgewebspunktaten von Typ-2-Diabetikern (n=13) und Nicht-Diabetikern (n=18). Die in der Literatur beschriebenen Unterschiede des IL-6-Spiegels im Blutplasma von Typ-2-Diabetikern und Nicht-Diabetikern können auch auf Ebene der Genexpression von *IL-6* in Fettgewebspunktaten von Typ-2-Diabetikern und Nicht-Diabetikern nachgewiesen werden (Fig. 11). Die *IL-6*-Expression beträgt in der Gruppe der Typ-2-Diabetiker 3,227 und in der Gruppe der Nicht-Diabetiker 2,329. Vergleicht man aber die Genexpression von *IL-6* von Individuen mit Typ-2-Diabetes oder Nicht-Diabetiker mit der *IL-6*-Expression des Gesamtpatientenkollektivs (s. Fig. 12), so zeigt sich überraschenderweise, dass Ausnahmen zwischen dem Zusammenhang zwischen IL-6-Level im Blutplasma und dem Typ-2-Diabetesstatus auftreten. Die Fig. 12 zeigt, dass die beiden weiblichen Typ-2-Diabetikerinnen Proband 1 und Proband 2 eine *IL-6*-Expression von 1,176 bzw. 2,133 aufweisen, die eher der *IL-6*-Expression der Gruppe der Nicht-Diabetiker mit 2,329 entspricht. Zudem zeigen die Nicht-Diabetiker Probanden 3 (männlich) und 4 (weiblich) eine *IL-6*-Expression von 4,063 bzw. 3,962, welche im Bereich der *IL-6*-Expression der Typ-2-Diabetiker-Gruppe mit 3,227 liegt. Diese Ergebnisse legen nahe, dass die niedrige *IL-6*-Expression bei den beiden Probandinnen mit Typ-2-Diabetes auf ein nicht entzündetes Fettgewebe hinweisen. Da IL-6 als inflammatorisches Zytokin mit Insulinresistenz im Zusammenhang steht (u.a. auch in der Leber durch Inhibition der Insulinwirkung), könnten bei niedrigen *IL-6* Expressionswerten therapeutische Maßnahmen zur Typ-2-Diabetes Behandlung, die die Insulinsekretion stärken, förderlich sein, wohingegen bei hohen *IL-6* Expressionswerten eher Maßnahmen zur Steigerung der Insulinsensitivität eingeleitet werden könnten.

**Beispiel 5**

**Datenanalyse mittels Selbstorganisierender Karten zeigt fünf unterschiedliche Cluster innerhalb des Patientenkollektivs**

**Material und Methoden**

**Probenvorbereitung**

Die Fettgewebsproben wurden während Operationen gewonnen wie in Beispiel 2 beschrieben. Auch die nachfolgenden Schritte der Probenaufbereitung, also RNA-Isolierung, cDNA-Synthese und die quantitative Real-Time-PCR erfolgten wie in Beispiel 2 beschrieben.

**Statistische Analyse**

Siehe oben, Methodik

**Ergebnis**

Dysfunktionales Fettgewebe bedingt u.a. eine Erhöhung des Risikos für mit Adipositas einhergehende Erkrankungen mit entzündlicher Komponente wie z.B. Typ-2-Diabetes. Eine länger anhaltende Freisetzung proinflammatorischer Zytokine, die zu einer subklinischen Entzündungsreaktion im jeweiligen Gewebe führt, kann unbehandelt eine T2D Erkrankung hervorrufen. Eines der bedeutendsten inflammatorischen Zytokine im Zusammenhang mit Adipositas und Diabetes ist IL-6, welches im Blutplasma bei den jeweiligen Erkrankten in erhöhten Konzentrationen vorliegt. Wie die Beispiele 2, 3 und 4 zeigen, sind niedrige *IL-6*-Expressionslevel aber auch bei Patienten mit Typ-2-Diabetes (T2D) und Übergewicht zu finden. Diese Erkenntnisse waren aufgrund der wissenschaftlichen Datenlage nicht zu erwarten und haben somit potentielle Auswirkungen auf eine Behandlung dieser Patienten. Um eine personalisierte Behandlung von z.B. Patienten mit T2D zu ermöglichen, ist es sinnvoll den speziellen Subtyp des T2D, an dem der Patient leidet, zu identifizieren. Ein spezieller T2D-Subtyp kann z.B. mit einer subklinischen Entzündungsreaktion im Fettgewebe in Verbindung gebracht werden, ein anderer Subtyp kann mit Problemen in der Insulinsekretion assoziiert sein. Bei diesen beiden T2D-Subtypen würden entsprechend ganz unterschiedliche Therapieansätze zum Einsatz kommen, um eine schnelle und zielgerichtete T2D-Behandlung zu gewährleisten. Um diese T2D-Subtypen zu identifizieren kann die Analyse der Beziehung der Biomarker *IL-6, HMGA2* und beispielsweise dem Alter mittels Kohonens Selbstorganisierenden Karten (SOM) helfen.

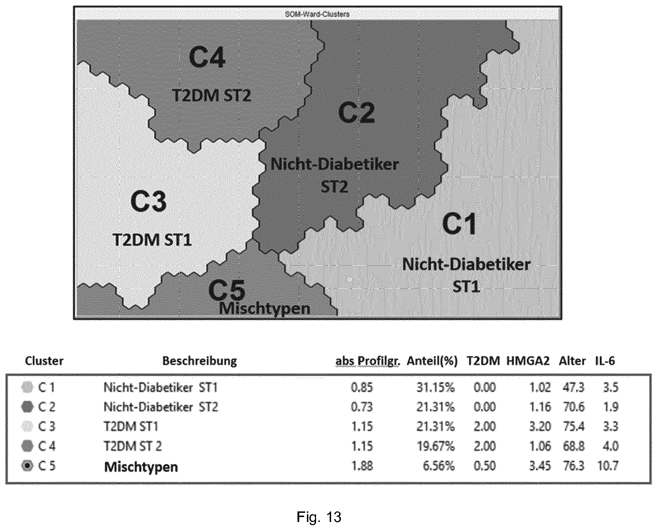

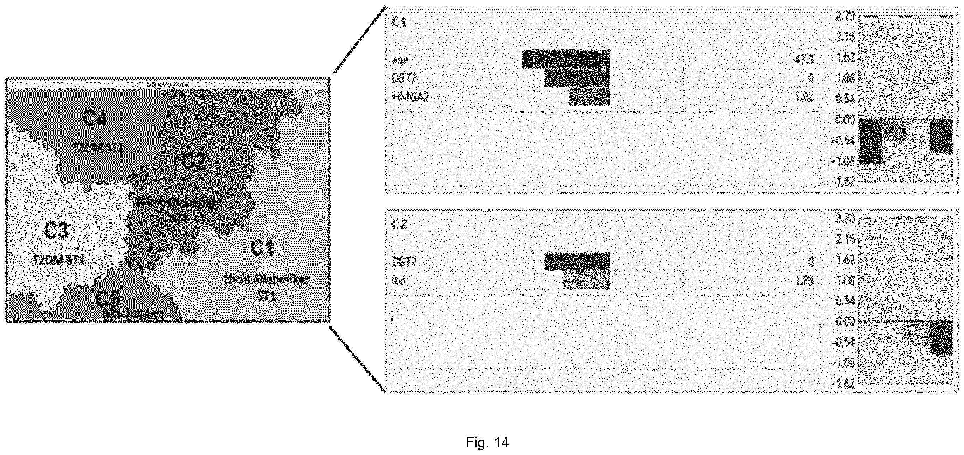

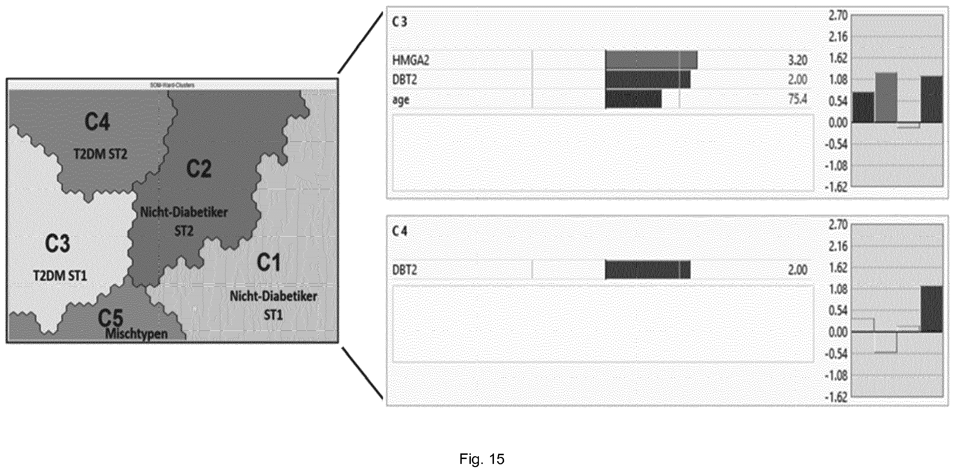

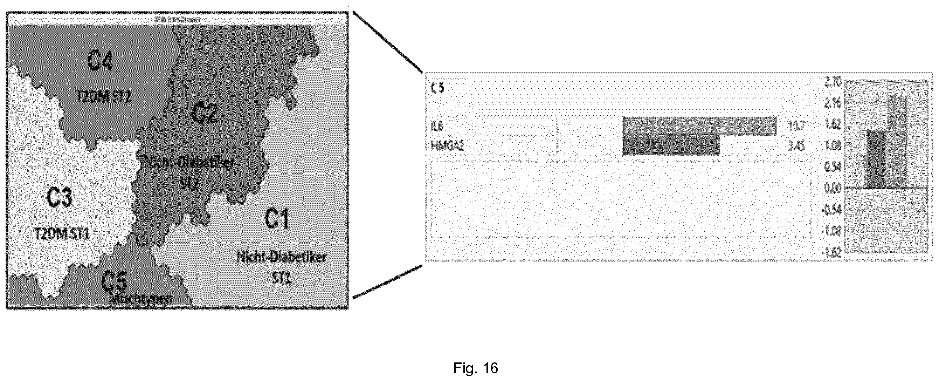

Die Fig. 13 zeigt nach Analyse der Biomarker *IL-6, HMGA2* und dem Alter (als Marker) mittels SOM überraschenderweise eine Einteilung des Patientenkollektivs in fünf Gruppen und nicht wie man z.B. nach einem HbA1c-Test erwarten würde in zwei Gruppen, nämlich Typ-2-Diabetiker und Nicht-Diabetiker. Die zugehörigen Daten finden sich in der Tabelle 2 und in den Figuren 14-16. Die Einteilung des Patientenkollektivs in fünf Gruppen überrascht auch insofern, da die Patentschrift DE10 2015 208 083 B3 eine Einteilung der Patienten in vier Gruppen offenbarte, nämlich zwei Übergewichtige- und zwei Normalgewichtige-Gruppen.

Die Datenanalyse mittels Selbstorganisierender Karten unterteilt das Probandenkollektiv unter Berücksichtigung der Parameter *HMGA2*- und *IL-6*-Expression in fünf Gruppen (Fig. 13). Dabei verteilen sich die Nicht-Diabetiker in zwei Cluster (C1 und C2). Die mittlere *HMGA2*-Expression in beiden Clustern ist gegenüber der mittleren *HMGA2*-Expression des Gesamtkollektivs erniedrigt. Die mittlere *IL-6*-Expression in Cluster C1 ist gegenüber dem Gesamtkollektiv nur leicht, in Cluster C2 deutlicher erniedrigt. Die Cluster C3 und C4 werden von Diabetikern gebildet. Die mittlere *HMGA2*-Expression ist in Cluster C3 stark erhöht, wohingegen *IL-6* hier im Mittel eine verringerte Expression aufweist. Im Cluster C4 liegt die mittlere *HMGA2*-Expression dagegen unter dem Mittelwert des Gesamtkollektivs, die mittlere *IL-6*-Expression ist leicht erhöht. Das fünfte Cluster besteht aus Mischtypen (C5). Wie auch im Diabetikercluster C3 liegt hier die mittlere *HMGA2*-Expression deutlich höher als der Mittelwert des Gesamtkollektivs, aber auch *IL-6* wird hier stark exprimiert und weist im Mittel sogar die stärkste Expression aller fünf Cluster auf.

**IL-6 Segmentation**

Tabelle 2

| Cluster | Bezeichnung | Mittelwert HMGA 2 | Abw. [%] | HMGA2 | Mittelwert IL-6 | Abw. [%] | IL-6 | Mittelwert Alter |
|---|---|---|---|---|---|---|---|---|
| C1 | Nicht-Diabetiker ST1 | 1.02 | -38.7 | erniedrigt | 3.5 | -6.4 | leicht erniedrigt | 47,3 |
| C2 | Nicht-Diabetiker ST2 | 1.16 | -30.3 | erniedrigt | 1.9 | -49.2 | erniedrigt | 70,6 |
| C3 | T2D ST1 | 3.2 | 92.4 | stark erhöht | 3.3 | -11.8 | erniedrigt | 75,4 |
| C4 | T2D ST2 | 1.06 | -36.3 | erniedrigt | 4 | 6.9 | leicht erhöht | 68,8 |
| C5 | Mischtype n | 3.45 | 107.4 | stark erhöht | 10.7 | 186.1 | stark erhöht | 76,3 |

Ohne an eine Theorie gebunden zu sein, gibt es folgende biologische Erklärungen für die Cluster:

C3 (T2D Subtyp 1): dysfunktionales Fettgewebe aufgrund vieler Präadipozyten (hoher *HMGA2*-Wert und verhältnismäßig niedriger/mittlerer *IL-6*-Wert): eher "Insulin-resistenter" T2D-Subtyp. Eine Differenzierung der Präadipozyten hin zu reifen Insulin-sensitiven Adipozyten wäre hier wünschenswert, es könnten Medikamente der Gruppe der Glitazone und evtl. Metformin hier helfen. Aber es gibt jedoch Hinweise, dass Insulin und Insulinähnliche Wachstumsfaktoren die Differenzierung von Präadipozyten in Richtung reifer Insulin-sensitive Adipozyten fördern (Ayoubi et al., 1999; Klemm et al., 2001). Daher ist es auch möglich Insulin und Insulinproduktion-fördernde Medikamente bei hohen *HMGA2*-Werten zu verordnen.

C4 (T2D Subtyp 2): funktionales Fettgewebe mit subklinischer Entzündungsreaktion (leicht erhöhte *IL-6*-Werte): eher ein generell "Insulin-sensitiver T2D-Subtyp" bzw. eine Insulinresistenz in der Leber aufgrund der leicht erhöhten *IL-6* Werte. Dieser T2D-Subtyp hat erwartbar ein Problem in der Insulin-Produktion/Sekretion in den ß-Zellen des Pankreas (sowie eine u.a. durch IL-6 vermittelte Inhibition der Insulinwirkung in der Leber), daher kann eine Behandlung hier auf die Verbesserung/Erhöhung der Insulin-Produktion abzielen (z.B. Sulfonylharnstoff und Glinide) und auf eine Verminderung der Entzündungsreaktion im Fettgewebe.

C5 ("Mischtyp"): dysfunktionales und entzündetes Fettgewebe aufgrund hoher *HMGA2*-Werte und sehr hohen *IL-6*-Werten: eher "Insulin-resistenter" Prä-T2D-/T2D-Subtyp. Hoher Anteil unreifer Insulin-resistenter Präadipozyten, die dadurch nicht in der Lage sind genügend Adipokine wie Adiponektin, die u.a. Körpergewebe sensitiver für Insulin machen, zu produzieren, sowie ein entzündetes Fettgewebe, das durch hohe IL-6 Konzentration letztlich für eine Insulinresistenz in der Leber sorgt. Patienten in dieser Gruppe leiden entweder schon an T2D oder sind gegebenenfalls schon Prä-Diabetiker, gekennzeichnet durch eine "starke" Insulin-Resistenz im Fettgewebe und gegebenenfalls in der Leber. Dies wird der Körper bei den "Prä-Diabetikern" durch eine Erhöhung der InsulinProduktion und -Sekretion versuchen zu kompensieren, bei den bekannten Diabetikern in dieser Gruppe ist die Insulin-Produktion hier schon zu stark gestört.

C1 (Nicht-Diabetiker Subtyp 1): spiegelt funktionales Fettgewebe mit einem relativen geringen Anteil an Präadipozyten sowie leicht erniedrigten Entzündungswerten wider. Es besteht das Risiko einer Entwicklung Richtung Gruppe C4.

C2 (Nicht-Diabetiker Subtyp 2): spiegelt funktionales Fettgewebe mit einem relativen geringen Anteil an Präadipozyten sowie niedrigen Entzündungswerten wider. Die im Vergleich zum Cluster C1 niedrigeren *IL-6*-Werte können im Zusammenhang mit dem Alter stehen.

**Patentansprüche**

1. Verfahren zur Prognose und/oder Diagnose einer Diabetes mellitus Typ II-Erkrankung, eines Probanden umfassend die Schritte:

a) Bestimmen des Genexpressionslevels des Genes *IL-6* in der Probe des Probanden und

b) Einordnen des Probanden unter Berücksichtigung des Genexpressionslevels des Genes *IL*- 6 in eine von wenigstens vier Risikogruppen,

wobei zum Einordnen des Probanden in Schritt b) ferner der Genexpressionslevel von *HMGA2,* berücksichtigt wird,

wobei als Gruppen für eine Einordnung in Schritt b) e die Gruppen mit der Markersituation

I) erniedrigtes relatives Genexpressionslevel für *IL-6*, stark erhöhtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq 60$, und

II) leicht erhöhtes relatives Genexpressionslevel für *IL-6*, erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq 60$, und

III) stark erhöhtes relatives Genexpressionslevel für *IL-6*, stark erhöhtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq 65$, und

IV) leicht erniedrigtes relatives Genexpressionslevel für *IL-6*, erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\leq 60$,

zur Verfügung stehen.

2. Verfahren nach Anspruch 1, wobei bei der Eingruppierung in Schritt b) ferner eines oder mehrere Merkmale des Probanden ausgewählt aus der Gruppe bestehend aus Alter, BMI, Größe, Gewicht, Geschlecht, Bauch- und Hüftumfang, Körperfettanteil, Muskelmasse, Total Body Water (TBW), Blutdruck, Raucherstatus, Bluthochdruck und Einnahme blutdrucksenkender Medikamente berücksichtigt wird.

3. Verfahren nach einem der Ansprüche 1 oder 2, wobei bei der Eingruppierung in Schritt b) ferner einer oder mehrere Blutwerte des Probanden ausgewählt aus der Gruppe bestehend aus Gesamtcholesterin, Triglyceride, HbA1c, HDL-Cholesterin, non-HDL-Cholesterin, LDL-Cholesterin, CRP, Blutzucker, Nüchternblutzucker, präprandialer Blutzucker, und postprandialer Blutzucker, berücksichtigt wird.

4. Verfahren nach einem der Ansprüche 1 bis 3, wobei die Probe aus Fettgewebe gewonnen wurde.

5. Verfahren nach Anspruch 4, wobei die Probe durch Punktion des subkutanen abdominalen Fettgewebes gewonnen wurde.

6. Verfahren nach einem der Ansprüche 1 bis 5, wobei der Proband ein Mensch ist.

7. Verfahren nach einem der Ansprüche 1 bis 6, wobei die Bestimmung des Genexpressionslevels auf mRNA-Ebene erfolgt.

8. Verfahren nach einem der Ansprüche 1 bis 7, wobei die Eingruppierung in Schritt b) unter Verwendung des multivariaten Modells der selbstorganisierenden Karten nach Kohonen erfolgt.

9. Verfahren nach einem der Ansprüche 1 bis 8, wobei die Einordnung in Schritt b) in eine von wenigstens 5 Gruppen erfolgt, wobei wenigstens zwei der Gruppen aus Individuen bestehen, die eine erhöhte Wahrscheinlichkeit der Ausbildung eines Diabetes mellitus Typ II besitzen und/oder wenigstens zwei der Gruppen aus Individuen bestehen, die Diabetes mellitus Typ II entwickelt haben.

10. Verfahren nach einem der Ansprüche 1 bis 9, wobei der Genexpressionslevel auf mRNA-Ebene relativ zu dem Genexpressionslevel eines Housekeeping-Gens gemessen wird.

11. Verfahren nach einem der Ansprüche 1 bis 10, wobei als mögliche Gruppe für eine Einordnung in Schritt b) die Gruppe mit der Markersituation
V) erniedrigtes relatives Genexpressionslevel für *IL-6*, erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\geq$ 50, zur Verfügung steht.

## Claims

1. Method for prognosing and/or diagnosing a type II diabetes mellitus disease of a subject, comprising the steps of:

   a) determining the gene expression level of the *IL-6* gene in the sample of the subject and
   b) classifying the subject into one of at least four risk groups, taking the gene expression level of the *IL-6* gene into account,
   wherein the gene expression level of HMGA2 is further taken into account for the classification of the subject in step b),
   wherein the groups with the marker situation

   I) lowered relative gene expression level for *IL-6*, greatly increased relative gene expression level for *HMGA2* and also at least one of the marker situations selected from the group consisting of increased HbAlc blood values, increased cholesterol values, increased triglycerides, lowered HDL cholesterol, increased non-HDL cholesterol, increased LDL cholesterol, increased CRP, increased blood sugar, increased fasting blood sugar, increased pre-prandial blood sugar, increased postprandial blood sugar and age $\geq$ 60, and
   II) slightly increased relative gene expression level for *IL-6*, lowered relative gene expression level for *HMGA2* and also at least one of the marker situations selected from the group consisting of increased HbAlc blood values, increased cholesterol values, increased triglycerides, lowered HDL cholesterol, increased non-HDL cholesterol, increased LDL cholesterol, increased CRP, increased blood sugar, increased fasting blood sugar, increased pre-prandial blood sugar, increased postprandial blood sugar and age $\geq$ 60, and
   III) greatly increased relative gene expression level for *IL-6*, greatly increased relative gene expression level for *HMGA2* and also at least one of the marker situations selected from the group consisting of increased HbAlc blood values, increased cholesterol values, increased triglycerides, lowered HDL cholesterol, increased non-HDL cholesterol, increased LDL cholesterol, increased CRP, increased blood sugar, increased fasting blood sugar, increased pre-prandial blood sugar, increased postprandial blood sugar and age $\geq$ 65, and
   IV) slightly lowered relative gene expression level for *IL-6*, lowered relative gene expression level for *HMGA2* and also at least one of the marker situations selected from the group consisting of non-increased HbAlc blood values, non-increased cholesterol values, non-increased triglycerides, increased HDL cholesterol, non-increased non-HDL cholesterol, non-increased LDL cholesterol, non-increased CRP, non-increased blood sugar, non-increased fasting blood sugar, non-increased pre-prandial blood sugar, non-increased postprandial blood sugar and age $\leq$ 60,

   are available as groups for a classification in step b).

2. Method according to Claim 1, wherein one or more features of the subject selected from the group consisting of age, BMI, height, weight, sex, abdominal and hip circumference, body fat percentage, muscle mass, total body water (TBW), blood pressure, smoker status, high blood pressure and ingestion of antihypertensive drugs are further taken into account in the classification in step b).

3. Method according to either of Claims 1 and 2, wherein one or more blood values of the subject selected from the group consisting of total cholesterol, triglycerides, HbAlc, HDL cholesterol, non-HDL cholesterol, LDL cholesterol, CRP, blood sugar, fasting blood sugar, pre-prandial blood sugar and postprandial blood sugar are further taken into account in the classification in step b).

4. Method according to any of Claims 1 to 3, wherein the sample was obtained from adipose tissue.

5. Method according to Claim 4, wherein the sample was obtained by puncture of subcutaneous abdominal adipose tissue.

6. Method according to any of Claims 1 to 5, wherein the subject is a human.

7. Method according to any of Claims 1 to 6, wherein the determination of the gene expression level is done at the mRNA level.

8. Method according to any of Claims 1 to 7, wherein the classification in step b) is done using the multivariate model of self-organizing maps by Korhonen.

9. Method according to any of Claims 1 to 8, wherein the classification in step b) is done into one of at least 5 groups, at least two of the groups consisting of individuals who have an increased probability of forming type II diabetes mellitus and/or at least two of the groups consisting of individuals who have developed type II diabetes mellitus.

10. Method according to any of Claims 1 to 9, wherein the gene expression level is measured at the mRNA level relative to the gene expression level of a housekeeping gene.

11. Method according to any of Claims 1 to 10, wherein the group with the marker situation

V) lowered relative gene expression level for *IL-6*, lowered relative gene expression level for *HMGA2* and also at least one of the marker situations selected from the group consisting of non-increased HbAlc blood values, non-increased cholesterol values, non-increased triglycerides, increased HDL cholesterol, non-increased non-HDL cholesterol, non-increased LDL cholesterol, non-increased CRP, non-increased blood sugar, non-increased fasting blood sugar, non-increased pre-prandial blood sugar, non-increased postprandial blood sugar and age $\geq 50$,
is available as a possible group for a classification in step b).

**Revendications**

1. Procédé pour le pronostic et/ou diagnostic d'une maladie du diabète sucré de type II d'un sujet comprenant les étapes de :

a) détermination du niveau d'expression de gène du gène IL-6 dans l'échantillon du sujet et
b) classement du sujet avec prise en compte du niveau d'expression de gène du gène IL-6 dans un d'au moins quatre groupes à risque,

dans lequel, pour classer le sujet dans l'étape b), le niveau d'expression de gène de HMGA2 est en outre pris en compte,
dans lequel les groupes avec la situation de marqueur

I) d'un niveau d'expression de gène pour IL-6 relatif bas, niveau d'expression de gène pour HMGA2 relatif fortement élevé ainsi qu'au moins une des situations de marqueur choisies dans le groupe constitué par des valeurs sanguines de HbA1c élevées, des valeurs de cholestérol élevées, des triglycérides élevés, un cholestérol HDL bas, un cholestérol non-HDL élevé, un cholestérol LDL élevé, une protéine C réactive élevée, une glycémie élevée, une glycémie à jeun élevée, une glycémie prépandiale élevée, une glycémie post-prandiale élevée et un âge $\geq 60$,
et

II) un niveau d'expression de gène pour IL-6 relatif légèrement élevé, un niveau d'expression de gène pour HMGA2 relatif bas ainsi qu'au moins une des situations de marqueur choisies dans le groupe constitué par des valeurs sanguines de HbA1c élevées, des valeurs de cholestérol élevées, des triglycérides élevés, un cholestérol HDL bas, un cholestérol non-HDL élevé, un cholestérol LDL élevé, une protéine C réactive élevée, une glycémie élevée, une glycémie à jeun élevée, une glycémie préprandiale élevée, une glycémie post-prandiale élevée et un âge $\geq 60$,
et

III) un niveau d'expression de gène pour IL-6 relatif fortement élevé, un niveau d'expression de gène pour HMGA2 relatif fortement élevé ainsi qu'au moins une des situations de marqueur choisies dans le groupe constitué par des valeurs sanguines de HbAlc élevées, des valeurs de cholestérol élevées, des triglycérides élevés, un cholestérol HDL bas, un cholestérol non-HDL élevé, un cholestérol LDL élevé, une protéine C réactive élevée, une glycémie élevée, une glycémie à jeun élevée, une glycémie prépandiale élevée, une glycémie postprandiale élevée et un âge $\geq 65$,
et

IV) un niveau d'expression de gène pour IL-6 relatif légèrement bas, un niveau d'expression de gène pour HMGA2 relatif bas ainsi qu'au moins une des situations de marqueur choisies dans le groupe constitué par des valeurs sanguines de HbAlc non élevées, des valeurs de cholestérol non élevées, des triglycérides non élevés, un cholestérol HDL élevé, un cholestérol non-HDL non élevé, un cholestérol LDL non élevé, une protéine C réactive non élevée, une glycémie non élevée, une glycémie à jeun non élevée, une glycémie prépandiale non élevée, une glycémie post-prandiale non élevée et un âge $\leq 60$,

sont mis à disposition comme groupes pour un classement dans l'étape b).

2. Procédé selon la revendication 1, dans lequel, lors de la classification dans l'étape b), une ou plusieurs caractéristiques du sujet choisies dans le groupe constitué par l'âge, l'IMC, la taille, le poids, le sexe, la circonférence abdominale et le tour de hanches, la masse adipeuse, la masse musculaire, la quantité totale d'eau dans le corps (TBW), la pression sanguine, le statut de fumeur, l'hypertension et la prise de médicaments antihypertenseurs est en outre prise en compte.

3. Procédé selon l'une quelconque des revendications 1 ou 2, dans lequel, lors de la classification dans l'étape b), une ou plusieurs valeurs sanguines du sujet choisies dans le groupe constitué par le cholestérol total, les triglycérides, le HbAlc, le cholestérol HDL, le cholestérol non-HDL, le cholestérol LDL, la protéine C réactive, la glycémie, la glycémie à jeun, la glycémie prépandiale, et la glycémie postprandiale, est en outre prise en compte.

4. Procédé selon l'une quelconque des revendications 1 à 3, dans lequel l'échantillon a été obtenu d'un tissu adipeux.

5. Procédé selon la revendication 4, dans lequel l'échantillon a été obtenu par ponction du tissu adipeux abdominal sous-cutané.

6. Procédé selon l'une quelconque des revendications 1 à 5, dans lequel le sujet est un humain.

7. Procédé selon l'une quelconque des revendications 1 à 6, dans lequel la détermination du niveau d'expression de gène s'effectue sur le plan de l'ARNm.

8. Procédé selon l'une quelconque des revendications 1 à 7, dans lequel la classification dans l'étape b) s'effectue à l'aide du modèle multi-variable des cartes auto-organisatrices de Kohonen.

9. Procédé selon l'une quelconque des revendications 1 à 8, dans lequel le classement dans l'étape b) s'effectue dans un d'au moins 5 groupes, dans lequel au moins deux des groupes sont constitués d'individus qui ont une forte probabilité de développement d'un diabète sucré de type II et/ou au moins deux des groupes sont constitués d'individus qui ont développé un diabète sucré de type II.

10. Procédé selon l'une quelconque des revendications 1 à 9, dans lequel le niveau d'expression de gène est mesuré sur le plan de l'ARNm par rapport au niveau d'expression de gène d'un gène domestique.

11. Procédé selon l'une quelconque des revendications 1 à 10, dans lequel, en tant que groupe possible pour un classement dans l'étape b), le groupe avec la situation de marqueur

V) un niveau d'expression de gène pour IL-6 relatif bas, un niveau d'expression de gène pour HMGA2 relatif bas ainsi qu'au moins une des situations de marqueur choisies dans le groupe constitué par des valeurs sanguines de HbA1c non élevées, des valeurs de cholestérol non élevées, des triglycérides non élevés, un cholestérol HDL élevé, un cholestérol non-HDL non élevé, un cholestérol LDL non élevé, une protéine C réactive non élevée, une glycémie non élevée, une glycémie à jeun non élevée, une glycémie prépandiale non élevée, une glycémie post-prandiale non élevée et un âge $\geq 50$,
est mis à disposition.

Fig. 1

$$w[j] := (w[j][1], w[j][2], ..., w[j][n])$$

$w[j][1]$  $w[j][2]$ .....  $w[j][n]$

$X_p[1]$   $x_p[2]$ ....   $X_p[n]$

$Input_p = (X_p[1], x_p[2], ..., X_p[n])$

Fig. 2

Fig. 3

Fig. 4

Fig. 5

Fig. 6

EP 3 714 068 B1

Fig. 7

Fig. 8

Fig. 9

Fig. 10

Fig. 11

Fig. 12

| Cluster | Beschreibung | abs Profilgr. | Anteil(%) | T2DM | HMGA2 | Alter | IL-6 |
|---|---|---|---|---|---|---|---|
| C 1 | Nicht-Diabetiker ST1 | 0.85 | 31.15% | 0.00 | 1.02 | 47.3 | 3.5 |
| C 2 | Nicht-Diabetiker ST2 | 0.73 | 21.31% | 0.00 | 1.16 | 70.6 | 1.9 |
| C 3 | T2DM ST1 | 1.15 | 21.31% | 2.00 | 3.20 | 75.4 | 3.3 |
| C 4 | T2DM ST 2 | 1.15 | 19.67% | 2.00 | 1.06 | 68.8 | 4.0 |
| C 5 | **Mischtypen** | 1.88 | 6.56% | 0.50 | 3.45 | 76.3 | 10.7 |

Fig. 13

Fig. 14

Fig. 15

Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015208083 B3

**In der Beschreibung aufgeführte Nicht-Patentliteratur**

- **MAUER J ; DENSON JL ; BRÜNING JC.** Versatile functions for IL-6 in metabolism and cancer. *Trends Immunol.,* 2015, vol. 36, 92-101
- **KERN PA ; RANGANATHAN S ; LI C ; WOOD L ; RANGANATHAN G.** Adipose tissue tumor necrosis factor and interleukin-6 expression in human obesity and insulin resistance. *Am J Physiol Endocrinol Metab,* 2001, vol. 280, E745-E751
- **OUCHI N ; PARKER JL ; LUGUS JJ ; WALSH K.** Adipokines in inflammation and metabolic disease. *Nat Rev Immunol.,* 2011, vol. 11, 85-97
- **SCHELLER J ; CHALARIS A ; SCHMIDT-ARRAS D ; ROSE-JOHN S.** The proand anti-inflammatory properties of the cytokine interleukin-6. *Biochim Biophys Acta,* 2011, vol. 1813, 878-888
- **ROSE-JOHN S.** IL-6 trans-signaling via the soluble IL-6 receptor: importance for the pro-inflammatory activities of IL-6. *Int J Biol Sci.,* 2012, vol. 8, 1237-1247
- **SABIO G ; DAS M ; MORA A ; ZHANG Z ; JUN JY ; KO HJ ; BARRETT T ; KIM JK ; DAVIS RJ.** A stress signaling pathway in adipose tissue regulates hepatic insulin resistance. *Science,* 2008, vol. 322, 1539-1543
- **ELLINGSGAARD H ; HAUSELMANN I ; SCHULER B ; HABIB AM ; BAGGIO LL ; MEIER DT ; EPPLER E ; BOUZAKRI K ; WUEEST S ; MULLER YD.** Interleukin-6 enhances insulin secretion by increasing glucagon-like peptide-1 secretion from L cells and alpha cells. *Nat Med.,* 2011, vol. 17, 1481-1489
- *Adipocyte,* 2014, vol. 3 (1), 39-45
- **HIRANO T ; YASUKAWA K ; HARADA H ; TAGA T ; WATANABE Y ; MATSUDA T ; KASHIWAMURA S ; NAKAJIMA K ; KOYAMA K ; IWAMATSU A et al.** Complementary DNA for a novel human interleukin (BSF-2) that induces B lymphocytes to produce immunoglobulin. *Nature,* 1986, vol. 324, 73-76
- **SZMITKO PE ; TEOH H ; STEWART DJ ; VERMA S.** Adiponectin and cardiovascular disease: state of the art?. *Am J Physiol Heart Circ Physiol.,* 2007, vol. 292, H1655-63
- **TEUVO KOHONEN.** Self-Organizing Maps. Springer-Verlag, 1995
- **QUINLAN, J. ROSS.** Induction of decision trees. *Machine Learning,* 1986, vol. 1 (1), 81-106
- **QUINLAN, J. ROSS.** C4.5. Programs for machine learning. *J. Ross Quinlan. San Mateo, Calif.: Morgan Kaufmann (The Morgan Kaufmann series in machine learning),* 1993
- **QUINLAN, J. ROSS.** Improved use of continuous attributes in C4. 5. *Journal of artificial intelligence research,* 1996, vol. 4, 77-90
- **HASTIE, T. ; TIBSHIRANI, R. ; FRIEDMAN, J. H.** The elements of statistical learning: Data mining, inference, and prediction. Springer Verlag, 2001
- **SONQUIST, J.A. ; MORGAN, J.N.** The Detection of Interaction Effects. Survey Research Center, Institute for Social Research, University of Michigan, 1964
- **VERGLEICHE SCHWARZ ; GIDEON E.** Estimating the dimension of a model. *Annals of Statistics,* 1978, vol. 6 (2), 461-464
- **KLEMKE M ; MEYER A ; HASHEMI NEZHAD M ; BEIGE G ; BARTNITZKE S ; BULLERDIEK J.** Loss of let-7 binding sites resulting from truncations of the 3' untranslated region of HMGA2 mRNA in uterine leiomyomas. *Cancer Genet Cytogenet,* 2010, vol. 196, 119-123
- **LIVAK KJ ; SCHMITTGEN TD.** Analysis of relative gene expression data using real-time quantitative PCR and the 2 Method. *Methods,* 2001, vol. 25, 402-408
- **MÖHLIG M ; BOEING H ; SPRANGER J ; OSTERHOFF M ; KROKE A ; FISHER E ; BERGMANN MM ; RISTOW M ; HOFFMANN K ; PFEIFFER AF.** Body mass index and C-174G interleukin-6 promoter polymorphism interact in predicting type 2 diabetes. *J Clin Endocrinol Metab.,* 2004, vol. 89, 1885-1890